# EUROPÄISCHE PATENTSCHRIFT

(11) **EP 2 805 129 B1**
(45) Veröffentlichungstag und Bekanntmachung des Hinweises auf die Patenterteilung: **08.07.2015**
(21) Anmeldenummer: 13702327.1
(22) Anmeldetag: 10.01.2013
(51) Int. Cl.: G01B 9/02, A61B 5/00

(54) **OPTISCHE KOHÄRENZTOMOGRAPHIE MIT ERWEITERTEM DYNAMIKBEREICH**
OPTICAL COHERENCE TOMOGRAPHY WITH AN EXTENDED DYNAMIC RANGE
TOMOGRAPHIE PAR COHÉRENCE OPTIQUE À PLAGE DYNAMIQUE ÉLARGIE

(30) Priorität: 16.01.2012 DE 102012000702
(43) Veröffentlichungstag der Anmeldung: 26.11.2014
(73) Patentinhaber: Karlsruher Institut Für Technologie (KIT), 76131 Karlsruhe (DE)
(72) Erfinder: SCHNEIDER, Simon, D-73642 Welzheim (DE); KOOS, Christian, 74936 Siegelsbach (DE); FREUDE, Wolfgang, 76199 Karlsruhe (DE); LEUTHOLD, Juerg, 8165 Oberweningen (DE)
(74) Vertreter: Müller-Boré & Partner Patentanwälte PartG mbB
(86) Internationale Anmeldenummer: PCT/EP2013/000061
(87) Internationale Veröffentlichungsnummer: WO 2013/107621

(56) Entgegenhaltungen:
- WO-A2-2011/068862
- US-A1- 2010 174 190
- CHANG S ET AL: "Attenuation compensation for optical coherence tomography imaging", OPTICS COMMUNICATIONS, NORTH-HOLLAND PUBLISHING CO. AMSTERDAM, NL, Bd. 282, Nr. 23, 1. Dezember 2009 (2009-12-01), Seiten 4503-4507, XP026694414, ISSN: 0030-4018, DOI: 10.1016/J.OPTCOM.2009.08.030 [gefunden am 2009-09-03]
- H. ISHIKAWA ET AL: "High Dynamic Range Imaging Concept-Based Signal Enhancement Method Reduced the Optical Coherence Tomography Measurement Variability", INVESTIGATIVE OPHTHALMOLOGY & VISUAL SCIENCE, Bd. 54, Nr. 1, 30. Januar 2013 (2013-01-30), Seiten 836-841, XP055060367, ISSN: 0146-0404, DOI: 10.1167/iovs.12-10990

## Beschreibung

Die Erfindung liegt auf dem Gebiet der optischen Messtechnik und betrifft die optische Kohärenztomographie (engl. Optical Coherence Tomography, OCT). Insbesondere betrifft die Erfindung eine Vorrichtung und ein Verfahren zur tiefenabhängigen Anpassung des Dynamikbereichs eines OCT-Systems an den zu messenden Verlauf der rückgestreuten Leistung. Damit lässt sich der Dynamikbereich des Messverfahrens vom Dynamikbereich eines verwendeten Analog-Digital-Umwandlers entkoppeln. Die Erfindung findet insbesondere Anwendung bei der Charakterisierung von stark streuenden oder stark absorbierenden biologischen oder technischen Proben.

Die optische Kohärenztomographie ist ein dreidimensional abbildendes Verfahren, das die optischen Streueigenschaften im Volumen einer Probe erfasst. OCT ermöglicht räumliche Auflösungen von wenigen Mikrometern in allen Raumrichtungen und zeichnet sich durch eine hohe Empfindlichkeit aus. Die Anwendungsbereiche der OCT liegen hauptsächlich im medizinischen Bereich und beinhalten beispielsweise Gewebeuntersuchungen in der Ophthalmologie, der Dermatologie und der Zahnheilkunde. Darüber hinaus findet OCT zunehmend Anwendung in der Materialwissenschaft sowie in der Verfahrenstechnik. OCT-Systeme existieren in verschiedenen Ausführungsformen. Weit verbreitete Ausführungsformen sind das Zeitbereichs-OCT (engl. time-domain-OCT, TD-OCT), welches erstmals von Huang et al. in "Optical Coherence Tomography", Science, vol. 254, no. 5035, 1178-1181, November 1991, beschrieben wurde, das Frequenzbereichs-OCT (engl. spectral-domain-OCT, SD-OCT), welches erstmals von Fercher et al. in "Measurement of intraocular distances by backscattering spectral interferometry", Elsevier Optics Communications, vol. 117, 42-48, Mai 1995, beschrieben wurde, und das OCT mit durchstimmbarer Laserquelle (engl. swept-source-OCT, SS-OCT), welches erstmals von Chinn et al. in "Optical coherence tomography using a frequency-tunable optical source", Optics Letters, vol. 22, no. 5, 340-342, März 1997, beschrieben wurde. Die hier beschriebene Erfindung bezieht sich insbesondere auf die Ausführungsformen TD-OCT und SS-OCT, auch wenn sie nicht grundsätzlich darauf beschränkt ist.

Bei der OCT wird kurzkohärentes Licht einer lateral einmodigen Quelle in die zu untersuchende Probe eingestrahlt. Ein Teil des von der Probe zurückgestreuten Lichts wird lateral einmodig erfasst mit dem Licht der Quelle zur Interferenz gebracht. Aus der zeitlichen Kohärenz des rückgestreuten und des emittierten Lichts wird das Rückstreuprofil entlang des anregenden Lichtstrahls ortsaufgelöst ermittelt. Eine dreidimensionale Darstellung erhält man, indem der Messstrahl an verschiedenen Punkten in die Probe eingestrahlt und jeweils Tiefenprofile der rückgestreuten Leistung (sog. A-Scans) aufgezeichnet werden.

Ein herkömmliches OCT-System besteht beispielsweise aus einem Michelson-Interferometer, das einen Beleuchtungsarm, einen Probenarm, einen Referenzarm und einen Detektionsarm umfasst. Jeder Arm kann dabei ein Lichtwellenleiter sein oder freistrahl-optisch aufgebaut sein. Der Beleuchtungsarm enthält eine Lichtquelle. Der Referenzarm umfasst einen optischen Pfad und einen Referenzreflektor. Der Probenarm umfasst einen optischen Pfad und einen fokussierenden Messkopf. Im Detektionsarm befindet sich ein Photodetektor. Wenn ein differentielles Empfangsprinzip Anwendung findet, erfüllt vorzugsweise der Beleuchtungsarm zusätzlich die Aufgabe eines zweiten Detektionsarmes. Die optische Leistung wird dafür mittels Koppler/Strahlteiler oder Zirkulator aus dem Beleuchtungsarm hin zum differentiellen Detektor ausgekoppelt.

Beim herkömmlichen TD-OCT wird eine breitbandig emittierende Lichtquelle (z.B. Weißlicht) verwendet. Als technische Realisierungen kommen beispielsweise Superlumineszenzdioden (SLD) in Frage oder Kurzpulslaser in Verbindung mit hochnichtlinearen, beispielsweise mikrostrukturierten Lichtleitfasern. Zur Erfassung der Kohärenzeigenschaften wird die Pfadlänge des Referenzarmes durch Verschieben des Referenzreflektors variiert. Rückstreuungen entlang des Messstrahles erzeugen dabei charakteristische Interferenzmuster, die als Funktion der Position des Referenzreflektors aufgezeichnet werden. Auf diese Art können Rückstreuungen aus unterschiedlichen Probentiefen gemessen werden. Ein so gewonnener Tiefenscan des Reflektivitätsprofils an einer Stelle in der Probe wird gemeinhin als "A-Scan" bezeichnet. Das dem A-Scan zugrundeliegende Interferenzmuster liegt in Form eines analogen elektrischen Signals am Ausgang des Photodetektors an. Es wird von einem Analog/Digital-Umsetzer ("AD-Wandler") erfasst und digital ausgewertet.

Ein herkömmliches SD-OCT-System weist ebenso wie das TD-OCT eine breitbandig emittierende Lichtquelle auf. Die Länge des Referenzarmes ist beim SD-OCT jedoch fest. Dafür wird das Interferenzlicht spektral getrennt detektiert und die entstehenden wellenlängenabhängigen Interferenzmuster werden ausgewertet. Dazu ist detektorseitig meist ein optisches Gitter angebracht, das die verschiedenen spektralen Anteile auf eine Detektorzeile abbildet. Die Leistungen auf den einzelnen Detektoren werden über einen AD-Wandler ausgelesen und digital weiterverarbeitet. Das tiefenaufgelöste Rückstreuintensitätsprofil (A-Scan) der Probe lässt sich mittels einer numerischen Fourier-Transformation berechnen.

Beim herkömmlichen SS-OCT wird als Lichtquelle ein schmalbandiger Laser verwendet, der schnell über ein großes optisches Frequenzband abgestimmt wird. Das Interferenzmuster wird dabei als Funktion der optischen Frequenz erfasst. Das so entstehende analoge elektrische Messsignal wird nach der Detektion über Analog/Digital-Wandlung erfasst und einer numerischen Fourier-Transformation unterzogen. Danach liegt ein tiefenaufgelöstes Rückstreuintensitätsprofil (A-Scan) der Probe vor.

Das interferometrische Messverfahren erlaubt eine hochempfindliche, kohärente Detektion des von der Probe zurückgestreuten Lichtes. Drexler et al. beschreiben in "In vivo ultrahigh-resolution optical coherence tomography", Optics Letters, Vol. 24, No. 17, 1221-1223, September 1999, ein Time-Domain OCT-System (TD-OCT) mit differenziellem Empfang, welches zwischen Empfänger und Datenerfassung mit einem Bandpassfilter und einem Verstärker ausgestattet ist. Das Filter ist als Butterworthfilter zweiter Ordnung ausgeführt. Es bewirkt eine Unterdrückung von Rauschanteilen im Signal und erlaubt damit eine Steigerung der Messempfindlichkeit.

Hofer et al. beschreiben in "Signal post processing in frequency domain OCT and OCM using a filter bank approach", Photonics West, Proc. SPIE Int. Soc. Opt. Eng. S. 644300-6, Januar 2007, ein Frequency-Domain OCT-System mit einem Digitalfilter, das dem Detektorarray nachgeschaltet ist. Das Filter dient der Kompensation des frequenzabhängigen Abfalls der Signalstärke, der durch die laterale Ausdehnung der Einzeldetektoren verursacht wird. Bei dieser Arbeit wird eine numerische Filterung betrieben, die einer A/D-Umsetzung nachgeschaltet ist. Bei all diesen Verfahren nimmt allerdings, insbesondere bei stark streuenden oder absorbierenden Proben, das Rückstreusignal mit der Tiefe oft stark ab. Der begrenzte Dynamikbereich herkömmlicher Messsysteme begrenzt somit auch die messbare Tiefe. Dies wird beispielsweise bei der Untersuchung von Proben zum Problem, bei denen das Rückstreusignal aus dem Volumen wesentlich schwächer ist als der von der Probenoberfläche herrührende Anteil. Kleine Signalanteile, die vornehmlich aus tieferen Schichten der Probe stammen, sind aufgrund eines begrenzten Dynamikbereichs nicht weiter detektierbar.

Um das Problem des hohen Dynamikbereichs von OCT-Signalen relativ zum beschränkten Dynamikbereich der Empfangselektronik zu beheben, schlägt das Patentdokument WO 2011/068862 einen logarithmisch arbeitenden Verstärker vor. In Chang et al. "Attenuation compensation for optical coherence tomographic imaging", in Optics Communications 282 (2009), p.4503 - 4507, wird eine digitale Nachbearbeitung vorgeschlagen, wohingegen in der nachveröffentlichten Schrifft Ishikawa et al. "High Dynamic Range Imaging Consept-Based Signal Enhancement Method Reduced the Optical Coherence Tomography Measurement Variability" in Investigative Ophthalmology & Visual Science (2013), Vol.54, No.1, die aus der Photographie bekannte Technik des "High Dynamic Range" auf OCT übertragen wird. In all diesen Verfahren ist jedoch die nutzbare Messtiefe und/oder Auflösung beschränkt.

Aufgabe der Erfindung ist es daher, die nutzbare Messtiefe der optischer Kohärenztomographie zu erweitern. Diese Aufgabe wird durch ein System und ein Verfahren zur optischen Kohärenztomographie sowie ein computer-implementiertes Verfahren und ein Computersystem zur Steuerung einer optischen Kohärenztomographie-Messung mit den in den unabhängigen Ansprüchen angegebenen Merkmalen gelöst. Bevorzugte Ausführungsformen sind Gegenstand der abhängigen Ansprüche.

Somit bietet die Erfindung in einem Aspekt ein System zur optischen Kohärenztomographie. Dieses System umfasst eine optische Detektionseinheit zur optischen Erfassung eines messtiefenabhängigen optischen Rückstreuprofils. Diese Detektionseinheit ist dabei insbesondere als interferometrische Einheit ausgestaltet, in der ein von einer Lichtquelle erzeugter Strahl in einen Probenstrahl und einen Referenzstrahl aufgeteilt wird, welche anschießend wieder zur Interferenz gebracht werden. Die optische Detektionseinheit ist ausgelegt, ein analoges Messsignal des erfassten messtiefenabhängigen optischen Rückstreuprofils zu generieren. Insbesondere wird das analoge optische Interferenzsignal mittels eines Photodetektors in ein analoges elektrisches Messsignal gewandelt.

Ein besonderer Aspekt der vorliegenden Erfindung ist nun, dass die optische Detektionseinheit eine steuerbare, also anpassbare (adaptive), analoge Signalverarbeitungseinheit umfasst, welche ausgelegt ist, in Abhängigkeit von einem erfassten Steuersignal eine selektive Gewichtung der zu verschiedenen Messtiefen gehörenden Anteile des analogen Messsignals vorzunehmen. Als Gewichtung wird dabei eine Verstärkung oder Abschwächung bestimmter Signalkomponenten im Messsignal im Vergleich zu anderen Signalkomponenten im Messsignal verstanden.

Je nach angewandtem Verfahren der optischen Kohärenztomographie werden die verschiedenen Messtiefen des Rückstreuprofils von unterschiedlichen Komponenten des analogen Messsignals repräsentiert. So repräsentiert beispielsweise bei einer TD-OCT-Messung das analoge Messsignal zu unterschiedlichen Zeitpunkten unterschiedliche Messtiefen, während beispielsweise bei einer SS-OCT-Messung verschiedene Frequenzkomponenten des analogen Messsignals unterschiedliche Messtiefen repräsentieren. Dementsprechend ist die adaptive analoge Signalverarbeitungseinheit entsprechend ausgelegt, beispielsweise das analoge Messsignal zeitlich selektiv zu gewichten (abzuschwächen oder zu verstärken) oder unterschiedliche Frequenzkomponenten gezielt selektiv zu gewichten. Die Auswahl und die Gewichtung der einzelnen Messtiefen wird dabei vorzugsweise über das Steuersignal eindeutig festgelegt. Das Steuersignal legt also die anpassbare Funktion der Signalverarbeitungseinheit fest. Dabei kann es sich um eine Auswahl aus einer Vielzahl diskreter vorgegebener Konfigurationen oder um eine Festlegung aus einer kontinuierlich veränderlichen Konfiguration handeln.

Außerdem umfasst das System eine Signalwandlereinheit, welche ausgelegt ist, das analoge Messsignal der optischen Detektionseinheit zu erfassen und in ein digitales Signal zu wandeln, welches damit auch als digitales Messsignal angesehen wird. Die Signalwandlereinheit fungiert somit als Analog-Digital-Wandler. Außerdem umfasst das System eine Datenerfassungs- und -verarbeitungseinheit, welches ausgelegt ist, Über- und/oder Unterschreitungen eines Dynamikbereichs der Signalwandlereinheit zu ermitteln. Die Datenerfassungs- und -verarbeitungseinheit ist außerdem ausgelegt, die analoge Signalverarbeitungseinheit mittels des Steuersignals derart zu steuern, dass der ermittelten Über- und/oder Unterschreitung des Dynamikbereichs der Signalwandlereinheit entgegengewirkt wird. Soweit also gewisse Signalkomponenten des analogen Messsignals aufgrund besonders starker oder besonders schwacher Rückstreuung einer zu untersuchenden Probe bei bestimmten Messtiefen außerhalb des Dynamikbereichs der Signalwandlereinheit liegen, werden diese Signalkomponenten von der adaptiven analogen Signalverarbeitungseinheit entsprechend gewichtet, also gedämpft oder verstärkt, um der ermittelten Über- oder Unterschreitung des Dynamikbereichs entgegenzuwirken. Vorzugsweise werden dabei also zu schwache Signalkomponenten verstärkt und/oder zu starke Signalkomponenten abgeschwächt.

Durch den Einfluss der Signalverarbeitungseinheit ergibt sich also ein modifiziertes analoges Messsignal, welches vorzugsweise innerhalb des Dynamikbereichs der Signalwandlereinheit liegt. Insbesondere weist das modifizierte analoge Messsignal vorzugsweise ein geringere Dynamik auf als das nicht modifizierte. Das modifizierte analoge Messsignal spiegelt somit auch nicht mehr genau die Rückstreucharakteristik der zu untersuchenden Probe wider, sondern ist um den Einfluss der Signalwandlereinheit verändert bzw. verfälscht, wobei diese Veränderung bzw. Verfälschung bekannt ist und insbesondere durch das Steuersignal gezielt gesteuert wird. Nach der Digitalisierung des modifizierten analogen Signals mittels der Signalwandlereinheit spiegelt somit auch das modifizierte digitale Signal nicht mehr exakt die Rückstreucharakteristik der zu untersuchenden Probe wider. Um dies zu kompensieren ist die Datenerfassungs- und -verarbeitungseinheit ausgelegt, eine Korrektur des von der Signalwandlereinheit erzeugten digitalen Signals derart vorzunehmen, dass dadurch die von der Signalverarbeitungseinheit vorgenommene Gewichtung verschiedener Signalanteile kompensiert wird. Nachdem der durch die Datenerfassungs- und -verarbeitungseinheit mittels des Steuersignal gesteuerte Einfluss der analogen Signalverarbeitungseinheit auf das analoge Messsignal bekannt ist, kann eine entsprechende Kompensation auf der digitalen Seite vorgenommen werden.

Es steht somit am Ende ein digitales Signal zur Verfügung das einem unverfälschten analogen Signal entspricht, also das sich aus einem analogen Signal ohne die analoge Signalverarbeitungseinheit im theoretischen Fall einer Signalwandlereinheit mit unbegrenzter Dynamik ergeben würde. Es können somit analoge Signale erfasst und in digitale Signale gewandelt werden, deren Dynamik größer ist als der Dynamikbereich der Signalwandlereinheit. Daraus ergibt sich eine Erweiterung der nutzbaren Messtiefe der optischen Kohärenztomographie selbst für Proben, welche an der Oberfläche stark reflektieren, und Proben, deren Rückstreuung mit der Tiefe stark abnimmt, welche also beispielsweise stark absorbieren.

Das der Erfindung zugrunde liegende Problem wird also durch die erfindungsgemäße Verwendung einer adaptiven analogen Signalverarbeitungseinheit gelöst, die eine Gewichtung der zu verschiedenen Tiefen gehörenden Anteile des analogen Messsignals erlaubt. Der Begriff des analogen Messsignals bezeichnet hierbei beispielsweise ein elektrisches Signal. Bei der Gewichtung kann es sich um eine Verstärkung oder eine Dämpfung der jeweiligen Signalanteile handeln. Diese kann, wie nachfolgend anhand von Beispielen bevorzugter Ausführungsformen noch genauer beschrieben wird, optisch oder analog-elektrisch erfolgen. Durch die Gewichtung wird der konstante Dynamikbereich des A/D-Umsetzers auf einen tiefenabhängig variablen Dynamikbereich für die Rückstreumessung abgebildet.

Durch die Verwendung einer adaptiven Signalverarbeitungseinheit kann somit beispielsweise für jede laterale Position einer zu untersuchenden Probe ein eigenes speziell angepasstes, also ortsabhängiges Empfindlichkeitsprofil entlang der vertikalen Messrichtung (Messtiefe) insbesondere automatisch realisiert werden.

In einer bevorzugten Ausführungsform umfasst die optische Detektionseinheit eine schmalbandige Lichtquelle (insbesondere eine durchstimmbare Laserquelle), deren Wellenlänge innerhalb eines Scan-Bereichs der optischen Detektionseinheit zeitlich veränderbar ist. Die spektrale Breite der Lichtquelle ist dabei also wesentlich kleiner als die spektrale Breite des Scan-Bereichs. Insbesondere handelt es sich bei dem System somit vorzugsweise um ein Swept-Source-OCT-System. Außerdem umfasst die optische Detektionseinheit vorzugsweise einen optischen Detektor, der ausgelegt ist, ein optisches Signal der optischen Detektionseinheit (insbesondere ein optisches Interferenzsignal) in ein analoges elektrisches Messsignal zu wandeln. In dieser Ausführungsform umfasst die analoge Signalverarbeitungseinheit vorzugsweise ein adaptives (insbesondere lineares) zeitinvariantes Filter, welches eine Filterung des elektrischen Messsignals vornimmt. Insbesondere wird damit eine Filterung des vom optischen Detektor erzeugten analogen elektrischen Messsignals vorgenommen, um das von der optischen Detektionseinheit generierte analoge Messsignal zu erzeugen.

Bei der adaptiven analogen Signalverarbeitungseinheit handelt es sich in dieser bevorzugten Ausführungsform somit um eine elektrische Signalverarbeitungseinheit. Sie ist dabei ausgelegt, eine spektral adaptive Filterung des elektrischen Signals des optischen Detektor (Photodetektors) vorzunehmen, also unterschiedliche Frequenzkomponenten unterschiedlich zu dämpfen oder zu verstärken und damit unterschiedlich zu gewichten. Dazu kann die Signalverarbeitungseinheit entsprechende passive und/oder aktive Filterkomponenten umfassen. Dabei ist die spektrale Filtereigenschaft der elektrischen Signalverarbeitungseinheit, also insbesondere die Auswahl und/oder Einstellung der Filterkomponenten mittels des Steuersignals steuerbar. Je nach Steuersignal kann also kontinuierlich oder diskret eine bestimmte spektrale Filtercharakteristik der Signalverarbeitungseinheit eingestellt werden, welche dann während der Messung über die gesamte zu untersuchende Messtiefe unverändert bleibt. Falls es bei einer Messung über die gewünschte bzw. prinzipiell erreichbare Messtiefe noch zu einer Überschreitung des Dynamikbereichs des A/D-Wandlers kommt, wird die Filtercharakteristik der Signalverarbeitungseinheit mittels eines entsprechenden Steuersignals vorzugsweise weiter angepasst.

Weiter bevorzugt umfasst das adaptive Filter ein Hochpassfilter (insbesondere nimmt die Transmission des Filters mit der Frequenz stetig zu) und/oder ein Bandpassfilter (insbesondere zur Selektion eines bestimmten Tiefenbereichs) und/oder einen Kerbfilter (insbesondere in Form eines Bandsperre zur Unterdrückung von starken Rückstreuspitzen an der Probenoberfläche) und/oder eine breitbandige Verstärkungseinheit (insbesondere um unter anderem die niedrigen Signalanteile bei großen Messtiefen stärker zu gewichten, während andere Signalanteile mittels weiterer Filterkomponenten gedämpft bzw. unterdrückt werden können). Dabei kann die Zusammenstellung und/oder die Frequenzbereiche und/oder das Ausmaß der Verstärkung bzw. Dämpfung in diskreten Stufen oder kontinuierlich durch das Steuersignal festgelegt sein.

In einer weiteren bevorzugten Ausführungsform umfasst die optische Detektionseinheit ein optisches Interferometer mit einem Probenarm und einem Referenzarm, wobei ein optischer Gangunterschied zwischen dem Probenarm und dem Referenzarm zur Abtastung der Messtiefe zeitlich veränderbar ist. Vorzugsweise nutzt das System insbesondere das Prinzip einer Time-Domain-OCT-Messung, bei der beispielsweise ein im Referenzarm vorgesehener Referenzreflektor zur Abtastung der Messtiefe in seiner Position in axialer Richtung des Referenzarmes variiert wird. Dabei umfasst die analoge Signalverarbeitungseinheit vorzugsweise ein zeitlich variables (vorzugsweise lineares) Verstärkungs- und/oder Dämpfungsglied, das von der Datenerfassungs- und -verarbeitungseinheit in Abhängigkeit vom (also synchron zum) optischen Gangunterschied zwischen dem Probenarm und dem Referenzarm (also insbesondere der Position des Referenzreflektors) gesteuert wird. Damit wird für jede Messtiefe eine eigene Gewichtung des Messsignals erreicht.

Die adaptive analoge Signalverarbeitungseinheit kann dabei auf verschiedene Weise implementiert sein. In einer bevorzugten Ausführungsform handelt es sich dabei um eine analoge elektrische Signalverarbeitungseinheit, die insbesondere direkt auf das von einem optischen Detektor (Photodetektor) erzeugte analoge elektrische Signal wirkt, bevor dies mittels der Signalwandlereinheit digitalisiert wird. Dazu umfasst die adaptive analoge Signalverarbeitungseinheit vorzugsweise ein elektrisches Verstärkungs- und/oder Dämpfungsglied.

In einer anderen bevorzugten Ausführungsform wirkt die adaptive analoge Signalverarbeitungseinheit nur indirekt auf ein von einem optischen Detektor (Photodetektor) erzeugtes analoge elektrische Signal, indem sie beispielsweise direkt auf zumindest einen Teil des optischen Signals wirkt bevor es vom optischen Detektor erfasst wird. In dieser bevorzugten Ausführungsform handelt es sich somit insbesondere um eine analoge optischen Signalverarbeitungseinheit, welche insbesondere ein optisches Verstärkungs- und/oder Dämpfungsglied umfasst.

Dabei kann das optische Verstärkungs- und/oder Dämpfungsglied an verschiedenen Stellen einer optischen Lichtführung (insbesondere Strahlführung) angeordnet sein und damit auf verschiedene Abschnitte der optischen Lichtführung wirken. So ist das optische Verstärkungs- und/oder Dämpfungsglied in einer bevorzugten Ausführungsform im Probenarm des optischen Interferometers angeordnet. In einer anderen bevorzugten Ausführungsform ist das optische Verstärkungs- und/oder Dämpfungsglied im Referenzarm des optischen Interferometers angeordnet. In einer weiteren bevorzugten Ausführungsform ist das optische Verstärkungs- und/oder Dämpfungsglied in einem Detektionsarm des optischen Interferometers angeordnet, in dem Licht vom Probenarm und vom Referenzarm interferieren.

In einem weiteren Aspekt stellt die Erfindung ein Verfahren zur optischen Kohärenztomographie bereit, welches ein Erfassen eines messtiefenabhänigen optischen Rückstreuprofils mittels einer optischen Detektionseinheit umfasst. Dabei wird ein analoges Messsignals des erfassten messtiefenabhänigen optischen Rückstreuprofils generiert. Dieses analoge Messsignal wird mittels einer Signalwandlereinheit in ein digitales Signal gewandelt. Außerdem umfasst das Verfahren ein Ermitteln einer Über- und/oder Unterschreitung eines Dynamikbereichs der Signalwandlereinheit und ein selektives Gewichten (Dämpfen und/oder Verstärken) der zu verschiedenen Messtiefen gehörenden Anteile des analogen Messsignals mittels einer adaptiven analogen Signalverarbeitungseinheit derart, dass einer ermittelten Über- und/oder Unterschreitung des Dynamikbereichs entgegengewirkt wird. Die Gewichtung erfolgt dadurch selektiv, dass der Einfluss auf unterschiedliche Signalkomponenten je nach ermittelter Über- und/oder Unterschreitung des Dynamikbereichs gezielt unterschiedlich sein kann, dass also einige Signalkomponenten mehr oder weniger geschwächt oder verstärkt werden, je nachdem welche Messtiefe sie repräsentieren.

Außerdem umfasst das Verfahren ein Korrigieren des digitalen Signals derart, dass dadurch die mittels der analogen Signalverarbeitungseinheit vorgenommene Gewichtung verschiedener Anteile des analogen Signals im digitalen Signal kompensiert wird.

Vorzugsweise wird das Erfassen des optischen Rückstreuprofils, das Generieren eines analogen Messsignals und das Ermitteln einer Über- und/oder Unterschreitung des Dynamikbereichs zunächst für eine erste Einstellung der adaptiven analogen Signalverarbeitungseinheit durchgeführt. Anschließend wird in Abhängigkeit von der erfassten Über- und/oder Unterschreitung des Dynamikbereichs eine angepasste Einstellung der adaptiven analogen Signalverarbeitungseinheit vorgenommen und das Erfassen des optischen Rückstreuprofils, das Generieren eines analogen Messsignals und das Ermitteln einer Über- und/oder Unterschreitung des Dynamikbereichs wird mit der angepassten Einstellung erneut durchgeführt. Soweit immer noch eine Über- und/oder Unterschreitung des Dynamikbereichs auftritt wird der Regelkreis vorzugsweise mit einer jeweils angepassten Einstellung der analogen Signalverarbeitungseinheit solange wiederholt, bis ein vorgegebenes Abbruchkriterium erfüllt wird. Anschließend erfolgt das Korrigieren des digitalen Signals gemäß der letzten Einstellung der analogen Signalverarbeitungseinheit.

Vorzugsweise wird also ein kurzkohärentes optisches Messverfahren zur hochdynamischen Erfassung eines ortsabhängigen Rückstreuprofils mit Hilfe einer optischen Mess- und Detektionseinheit, die ein analoges Messsignal abgibt, eines Analog-Digital-Umsetzers, einer adaptiven analogen Signalverarbeitungseinheit und einer Datenerfassungs- und -verarbeitungseinheit bereitgestellt, welches folgende Schritte umfasst:
1. Aufnahme der Messdaten eines Rückstreuprofils mit einer ersten Einstellung der adaptiven analogen Signalverarbeitungseinheit,
2. Auswertung der Messdaten durch die Datenerfassungs- und -verarbeitungseinheit,
3. Analyse der Daten hinsichtlich Überschreitung der oberen (Überschreitung) und unteren (Unterschreitung) Grenzen des Dynamikbereichs,
4. Ableitung einer angepassten Signalverarbeitungsvorschrift für die adaptive analoge Signalverarbeitungseinheit,
5. Aufnahme der Messdaten eines Rückstreuprofils mit der angepassten Einstellung der adaptiven analogen Signalverarbeitungseinheit,
6. Iterative Wiederholung der Schritte 2-5 bis ein vorgegebenes Abbruchkriterium erfüllt wird,
7. Numerische (digitale) Kompensation des Einflusses der adaptiven analogen Signalverarbeitung auf die Messdaten,
8. Ausgabe des hochdynamisch erfassten ortsabhängigen Rückstreuprofils.

In einem weiteren Aspekt bietet die Erfindung ein computerimplementiertes Verfahren zur Steuerung einer optischen Kohärenztomographie-Messung, umfassend
- Erfassen eines von einer Signalwandlereinheit erzeugten digitalen Signals (digitales Messsignal) eines messtiefenabhängigen Rückstreuprofils einer optischen Kohärenztomographie-Messung;
- Ermitteln einer Über- und/oder Unterschreitung eines Dynamikbereichs der Signalwandlereinheit (z.B. durch Aufeinanderfolge einer vorgegebenen Mindestzahl an digitalen Werten in einem vorgegebenen Wertebereich);
- Generieren eines Steuersignals zur Steuerung einer adaptiven analogen Signalverarbeitungseinheit derart, dass eine durch das Steuersignal von der adaptiven analogen Signalverarbeitungseinheit bewirkte selektive Gewichtung von zu verschiedenen Messtiefen gehörenden analogen Signalanteilen des Rückstreuprofils der optischen Kohärenztomographie-Messung der ermittelten Über- und/oder Unterschreitung des Dynamikbereichs entgegenwirkt; und
- Korrigieren des digitalen Signals derart, dass dadurch die von der Signalverarbeitungseinheit vorgenommene selektive Gewichtung verschiedener Signalanteile kompensiert wird.

Um ein geeignetes Steuersignal zu generieren, wird vorzugsweise zunächst eine computerles- und -verarbeitbare Steuervorschrift bereitgestellt, die einen Zusammenhang zwischen Über- und/oder Unterschreitungen des Dynamikbereichs und dem zu generierenden Steuersignal festlegt (z.B. in Form einer Formel). Dieser vorbestimmte und hinterlegte Zusammenhang wird auf eine ermittelte Über- und/oder Unterschreitung des Dynamikbereichs angewandt und liefert als Resultat das zu generierende Steuersignal. Der hinterlegte Zusammenhang spiegelt dabei die optischen bzw. elektrischen Dämpfungs- und/oder Verstärkungseigenschaften der analogen Signalverarbeitungseinheit sowie deren Abhängigkeit vom Steuersignal wider. Soll also beispielsweise eine bestimmte Frequenz oder ein bestimmter Frequenzbereich des analogen Messsignals gedämpft oder verstärkt werden, um eine bestimmte Verletzung des Dynamikbereichs zu vermeiden, legt die Steuervorschrift ein dafür geeignetes Steuersignal fest. Insbesondere wird die Steuervorschrift in einer Datenbank hinterlegt.

In einem weiteren Aspekt bietet die Erfindung ein Computersystem zur Steuerung einer optischen Kohärenztomographie-Messung, umfassend:
- einen digitalen Signaleingang zum Erfassen eines von einer Signalwandlereinheit erzeugten digitalen Signals (digitales Messsignal) eines messtiefenabhängigen Rückstreuprofils einer optischen Kohärenztomographie-Messung;
- ein Datenauswertungsmodul zum Ermitteln einer Über- und/oder Unterschreitung eines Dynamikbereichs der Signalwandlereinheit;
- einen Datenspeicher mit einer Steuervorschrift, welche für Über- und/oder Unterschreitungen des Dynamikbereichs der Signalwandlereinheit Werte eines Steuersignals zur Steuerung einer adaptiven analogen Signalverarbeitungseinheit derart festlegt, dass eine durch das Steuersignal von der adaptiven analogen Signalverarbeitungseinheit bewirkte selektive Gewichtung von zu verschiedenen Messtiefen gehörenden analogen Signalanteile des Rückstreuprofils der optischen Kohärenztomographie-Messung den Über- und/oder Unterschreitungen des Dynamikbereichs entgegenwirkt; und
- ein Steuermodul, welches der ermittelten Über- und/oder Unterschreitung des Dynamikbereichs gemäß der im Datenspeicher hinterlegten Steuervorschrift ein Steuersignal generiert,
wobei das Datenauswertungsmodul ausgelegt ist, das digitale Signal derart zu korrigieren, dass dadurch die gemäß der Steuervorschrift für das erzeugte Steuersignal von der Signalverarbeitungseinheit bewirkte selektive Gewichtung verschiedener Signalanteile kompensiert wird.

Vorzugsweise ist das Computersystem ausgelegt eines der hier beschriebenen Verfahren auszuführen bzw. zu steuern. In einer bevorzugten Ausführungsform eines erfindungsgemäßen Systems zur optischen Kohärenztomographie umfasst die Datenerfassungs- und -verarbeitungseinheit ein Computersystem zur Steuerung einer optischen Kohärenztomographie-Messung gemäß der Erfindung, insbesondere gemäß einer der hier beschriebenen bevorzugten Ausführungsformen.

Bevorzugte Ausführungsformen der Erfindung werden nachfolgend unter Bezugnahme auf die beigefügten Zeichnungen beispielhaft erläutert. Dabei zeigen:
- Fig. 1A-1D: beispielhafte Signalverläufe zur Veranschaulichung einer bevorzugten Wirkung eines erfindungsgemäßen Verfahrens aufgrund eines Vergleichs mit herkömmlichen Signalverläufen;
- Fig. 2: einen schematischen Aufbau eines Systems gemäß einer ersten bevorzugten Ausführungsform der Erfindung;
- Fig. 3: einen schematischen Aufbau eines Systems gemäß einer zweiten bevorzugten Ausführungsform der Erfindung;
- Fig. 4: einen schematischen Aufbau eines Systems gemäß einer dritten bevorzugten Ausführungsform der Erfindung;
- Fig. 5: einen schematischen Aufbau eines Systems gemäß einer vierten bevorzugten Ausführungsform der Erfindung;
- Fig. 6: einen schematischen Aufbau eines Systems gemäß einer fünften bevorzugten Ausführungsform der Erfindung;
- Fig. 7: ein Flussdiagramm zu Veranschaulichung eines Verfahrens gemäß einer bevorzugten Ausführungsform der Erfindung;
- Fig. 8: einen schematischen Aufbau eines Systems gemäß einer sechsten bevorzugten Ausführungsform der Erfindung;
- Fig. 9: einen schematischen Aufbau eines Systems gemäß einer siebten bevorzugten Ausführungsform der Erfindung.

Beim TD-OCT ist die beobachtete Rückstreutiefe durch die Position des Referenzreflektors bestimmt. Die erfindungsgemäße Gewichtung des analogen Messsignals ist dabei vorzugsweise an die Position des Referenzreflektors gekoppelt. Sie kann beispielsweise durch ein zeitlich variables elektrisches Verstärkungs- oder Dämpfungsglied erfolgen. Alternativ oder zusätzlich können zeitlich variable optische Verstärkungs- oder Dämpfungsglieder zum Einsatz kommen.

Beim SS-OCT werden Streulichtanteile aus verschiedenen Tiefen auf verschiedene Frequenzkomponenten des elektrischen Messsignals abgebildet. Eine tiefenselektive Gewichtung des Messsignals kann also beispielsweise durch eine Filterung des analog-elektrischen Signals mit einem linearen zeitinvarianten Filter (linear, time invariant, LTI) erreicht werden.

Bei beiden Verfahren werden die Einflüsse der analogen Signalverarbeitung in einem digitalen Verarbeitungsschritt wieder korrigiert. In einem iterativen Verfahren wird vorzugsweise die Gewichtungsvorschrift (Signalverarbeitungsvorschrift) an die Form des zu messenden Rückstreuprofils angepasst. Damit lassen sich Rückstreuprofile erfassen, deren Leistungsvariationen größer sind als der Dynamikbereich des A/D-Wandlers.

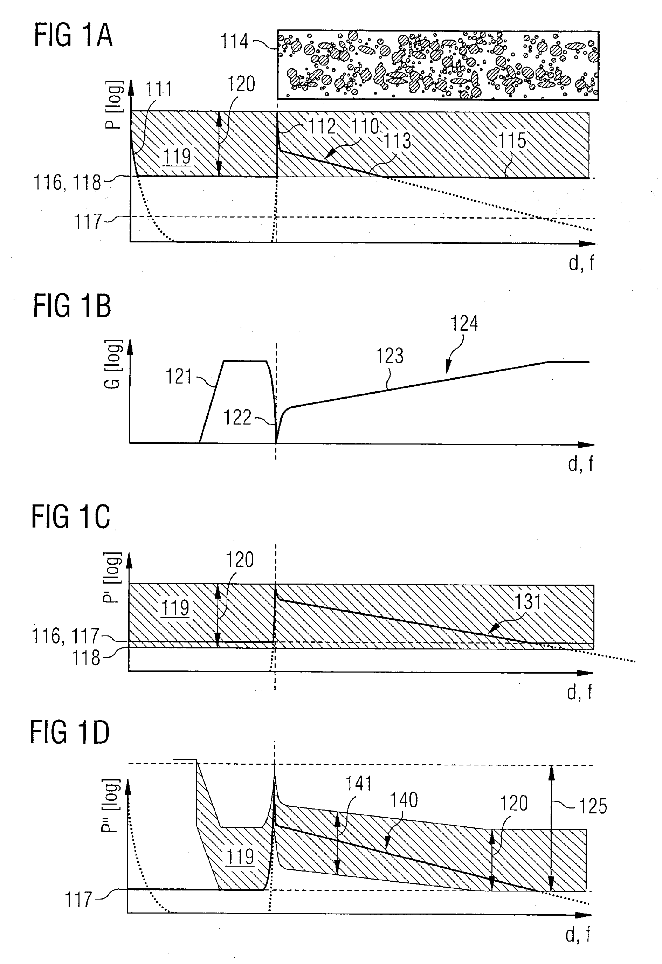

In **Fig. 1A** bis **Fig. 1D** wird die Funktionsweise des Messsystems und das Messverfahren anhand einer bevorzugten Ausführungsform erläutert. Dabei ist ein beispielhafter A-Scan 110 als logarithmischer Verlauf des Rückstreuprofils P über der Tiefe d dargestellt, die bei SS-OCT-Systemen direkt mit der Überlagerungsfrequenz f der jeweiligen spektralen Komponente im Interferenzsignal verknüpft ist. Dieses Rückstreuprofil P setzt sich aus verschiedenen Signalbereichen zusammen: Meist tritt eine starke Signalspitze 112 an der Position der Probenoberfläche auf, gefolgt von einem mit zunehmender Eindringtiefe in die Probe 114 exponentiell abfallenden Verlauf der Rückstreuung 113, die bei größeren Probentiefen die Untergrenze 116 des Messbereiches erreicht, was zu einer Signaluntergrenze 115 führt, die die tatsächliche Charakteristik der mit steigender Probentiefe (Messtiefe) weiter abnehmenden Signalintensität nicht mehr widerspiegelt. Im Falle von SS-OCT führen niederfrequente Anteile und Gleichanteile im Interferenzsignal zu einem starken Anstieg 111 des rekonstruierten Rückstreuprofils am linken Rand des Messbereiches.

Bei der herkömmlichen Aufnahme eines A-Scans ist die Untergrenze 116 des Messbereiches 119 im allgemeinen durch die stärkste innerhalb des A-Scans auftretende Rückstreuung und die Quantisierungstiefe des A/D-Wandlers bestimmt: Das maximale Rückstreusignal muss noch innerhalb des Quantisierungsbereiches des A/D-Wandlers liegen. Bei vorgegebenem Dynamikbereich 120 des A/D-Wandlers (typischerweise < 72 dB) ist damit auch der Pegel 118 des (numerischen) Quantisierungsrauschens vorgegeben. Schwache Anteile der rückgestreuten Leistung aus tiefen Bereichen der Probe werden dadurch vom Quantisierungsrauschen 118 des A/D-Wandlers überdeckt, obwohl eine durch das Rauschen des Analogsignals definierte prinzipielle Empfindlichkeitsgrenze 117 des Systems noch nicht erreicht ist. Das Rauschen des Analogsignals beinhaltet insbesondere Schrotrauschen des optischen Detektionsprozesses und/oder thermisches Rauschen der analogen Elektronik und bestimmt die prinzipiell erreichbare Empfindlichkeit des Systems.

Entsprechend dieser Erfindung findet nun durch eine analoge Signalverarbeitungseinheit eine definierte Manipulation des analogen Messsignals statt, die zu einer tiefenselektiven Gewichtung führt. Diese Gewichtung kann linear oder nichtlinear sein. So kann es sich dabei insbesondere um eine Verstärkung für größere Tiefen (größere Abstände von der Oberfläche) oder eine Abschwächung für geringere Tiefen (nahe der Oberfläche) handeln. Im Allgemeinen wird eine Gewichtungsvorschrift 124 insbesondere so gewählt, dass Signalanteile aus schwach streuenden Bereichen gegenüber solchen aus stark streuenden Abschnitten verstärkt werden.

Bei einer Realisierung als TD-OCT-System lässt sich die Charakteristik mittels eines zeitlich variablen Verstärkungs- oder Dämpfungsgliedes erreichen. Bei einer Realisierung als SS-OCT-System können verschiedene spektrale Filter in Form adaptiver linearer zeitinvarianter Systeme zur Anwendung kommen. Der Verlauf der Gewichtungsvorschrift 124 über der Tiefe d verhält sich dabei vorzugsweise im Wesentlichen invers zum erwarteten Verlauf des Rückstreuprofils. Fig. 1 B veranschaulicht ein Beispiel einer Gewichtungsvorschrift G(d) gemäß einer bevorzugten Ausführungsform. Im gezeichneten Beispiel enthält das Filter einen Hochpassbereich 121, ein Kerbfilter 122 und eine exponentiell mit der Frequenz ansteigende Hochpasscharakteristik 123.

Durch die ggf. iterative Optimierung der Signalverarbeitungsvorschrift für das analoge Messsignal und die damit verbundene Anpassung der tiefenabhängigen Gewichtungsvorschrift G(d) wird ein modifiziertes Rückstreuprofil P' 131 erzeugt (Fig. 1 C), das von Störgrößen (z.B. dem Anstieg 111 an der linken Seite des Messbereiches) befreit ist und dessen Verlauf in den verfügbaren Dynamikbereich 119 des A/D-Wandlers eingepasst ist. Durch die damit verbundene Nivellierung der Signalpegel innerhalb des A-Scans kann der Pegel 118 des (numerischen) Quantisierungsrauschens unter die prinzipielle Rauschgrenze 117 des analogen Systems abgesenkt werden. Die Untergrenze 116 des Messbereiches ist in diesem Fall durch die prinzipielle Empfindlichkeitsgrenze 117 des Messsystems gegeben.

Mit diesem Verfahren können also A-Scans aufgenommen werden, in denen sehr starke und sehr schwache Streuleistungen gleichermaßen auftreten. Die durch die analoge Signalverarbeitung verursachte Modifikation des Rückstreuprofils wird anschließend in der digitalen Phase numerisch wieder kompensiert. Bei dieser Korrektur wurden systembedingte Störgrößen wie z.B. dem Anstieg 111 an der linken Seite des Messbereiches eines SS-OCT eliminiert. In Fig. 1D bezeichnet P" eine beispielhafte Rekonstruktion des tatsächlichen Rückstreuprofils 140 nach der numerischen Korrektur der tiefenabhängigen Gewichtung.

Bei dieser Rekonstruktion handelt es sich um eine eineindeutige Abbildungsvorschrift, die den Messbereich 119 des A/D-Wandlers auf einen tiefenabhängigen Messbereich 141 des Gesamtsystems abbildet. Innerhalb dieses Messbereiches können Rückstreuprofile mit einem Dynamikbereich 125 erfasst werden, der nicht mehr durch die Dynamikbereich 120 des A/D-Wandlers begrenzt ist.

Der Vorteil dieser Erfindung besteht darin, dass durch die tiefenselektive Gewichtung des analogen Messsignals ein tiefenabhängiger Verlauf des erfassbaren Messbereiches definiert werden kann, der an die Form des zu messenden Rückstreuprofils (110, 140) angepasst wird. Der Begriff "Rückstreuprofil" bezeichnet dabei den Verlauf der vom System detektierten rückgestreuten optischen Leistung P als Funktion der Tiefe d. Durch die tiefenselektive Gewichtung lassen sich Rückstreuprofile erfassen, deren Leistungsvariationen größer sind als der Dynamikbereich (120) des A/D-Wandlers. Die Erfindung ermöglicht es damit, den vollen Empfindlichkeitsbereich eines OCT-Systems zu nutzen und somit Streuereignisse in stark streuenden und absorbierenden Medien in großer Tiefe zu detektieren.

Dadurch lassen sich beispielsweise rückgestreute Signalanteile erfassen, die in der Probe gegenüber dem eingestrahlten Licht Abschwächungen von mehr als 120 dB erfahren haben. Der erfassbare Dynamikbereich der Streulichtmessung wird unter anderem durch die effektive Quantisierungstiefe des AD-Wandlers bestimmt, die typischerweise zwischen 9 und 12 bit liegt und Rauschen und harmonische Verzerrungen im Wandler einbezieht. Dies entspricht einem Dynamikbereich zwischen 58 und 72 dB. Innerhalb eines A-Scans entspricht dies dem Verhältnis der maximalen zur minimalen Signalamplitude.

Im Folgenden werden bevorzugte Ausführungsformen erfindungsgemäßer Systeme anhand bevorzugter Implementierungen als TD-OCT-Systeme und SS-OCT-Systeme getrennt beschrieben. Ein entsprechendes Verfahren ist dabei auf beide möglicher Fälle beispielhafter Implementierungen in analoger Weise anwendbar.

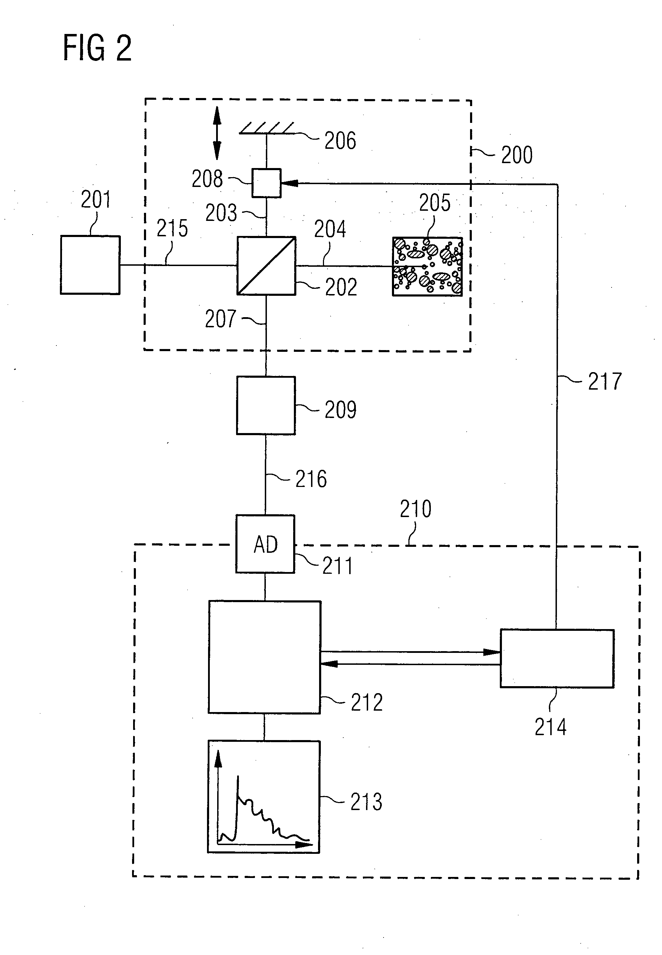

Beim TD-OCT wird die erfindungsgemäße Gewichtung des analogen Messsignals vorzugsweise an die Position des Referenzreflektors gekoppelt. Dies wird beispielsweise in einer oder mehrere der nachfolgend beschriebenen bevorzugten Anordnungen implementiert. Die nachfolgend beschriebenen bevorzugten Ausführungsform (**Fig. 2** bis **Fig. 4**) legen ein OCT-System auf Basis eines Michelson-Interferometers 200 zugrunde, wobei die Erfindung aber auch in anderen Interferometer-Anordnungen realisiert werden kann.

Licht einer in einem Beleuchtungsarm 215 angeordneten breitbandigen Lichtquelle 201, insbesondere einer Weißlichtquelle, wird von einem Strahlteiler 202 auf einen Referenzarm 203 und einen Probenarm 204 aufgeteilt, wobei im Probenarm eine zu untersuchende Probe 205 angeordnet werden kann. Im Referenzarm 203 ist ein Referenzreflektor 206 angeordnet, der das auftreffende Referenzlicht wieder zum Strahlteiler 202 reflektiert. Die Position des Referenzreflektors ist dabei derart entlang der Strahlrichtung des Referenzlichtes kontrolliert veränderbar, dass damit der optische Gangunterschied zwischen dem im Referenzarm 203 geführten Referenzlicht und dem im Probenarm 204 geführten Probenlicht veränderbar ist.

Rückgestreutes Licht von der Probe 205 interferiert im Strahlteiler 202 und in einem Detektionsarm 207 des Systems mit vom Referenzreflektor 206 rückreflektiertem Licht und trifft anschließend auf einen Detektor 209 (Photodetektor), der das optische Signal vorzugsweise in ein elektrische Signal in Form eines analogen Messsignals 216 umwandelt.

Zur digitalen Weiterverarbeitung, Speicherung und Darstellung der Messergebnisse umfasst das Messsystem eine Datenerfassungs- und -verarbeitungseinheit 210, welche ausgelegt ist, das analoge Messsignal 216 (insbesondere als Spannungssignal) mittels eines Analog/Digital-Umsetzers (A/D) 211 zu erfassen und in quantisierter Form zu digitalisieren. Der A/D-Umsetzer 211 besitzt vorzugsweise eine Quantisierungstiefe im Bereich von etwa 8 bit bis etwa 16 bit, besonders bevorzugt im Bereich von etwa 12 bit bis etwa 16 bit. In der Datenerfassungs- und -verarbeitungseinheit 210 wird das digitale Signal anschließend mittels eines Datenauswertungsmoduls 212 digital aufbereitet, um es schließlich über eine Datenausgabeeinheit 213 (z.B. in Form eines Bildschirms) ausgeben zu können.

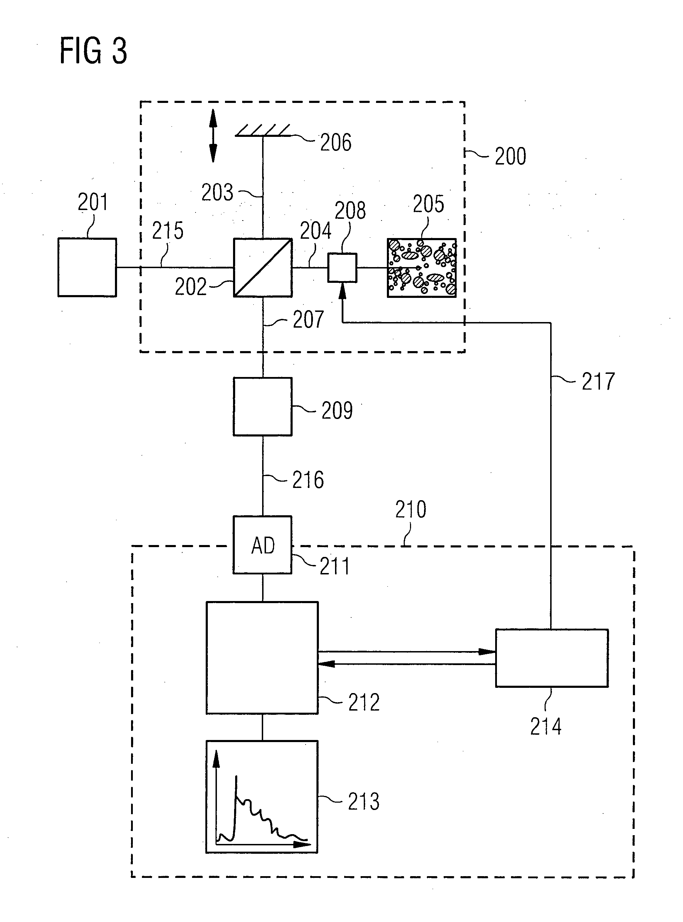

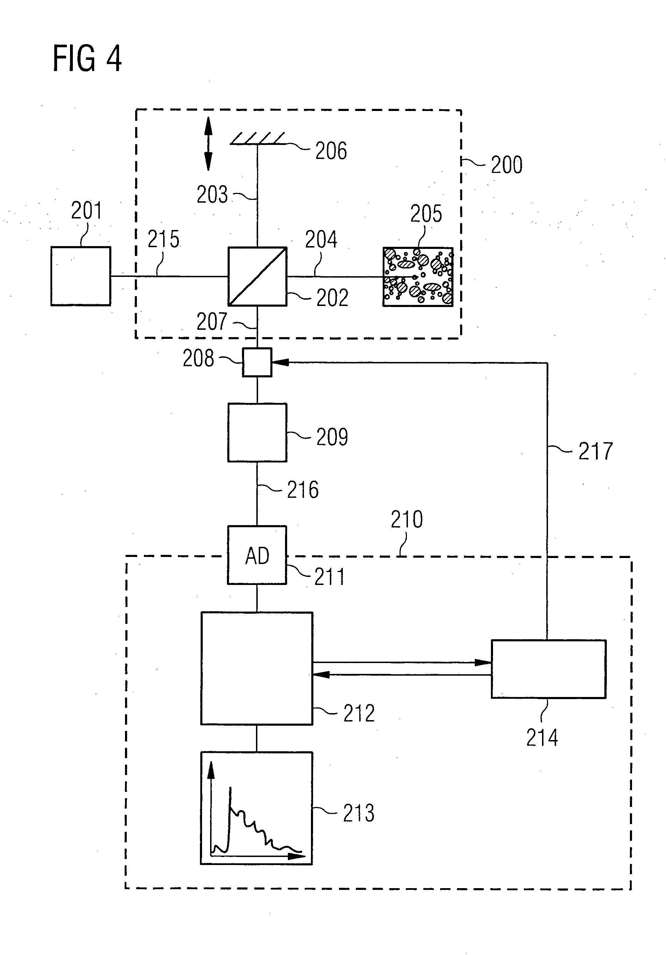

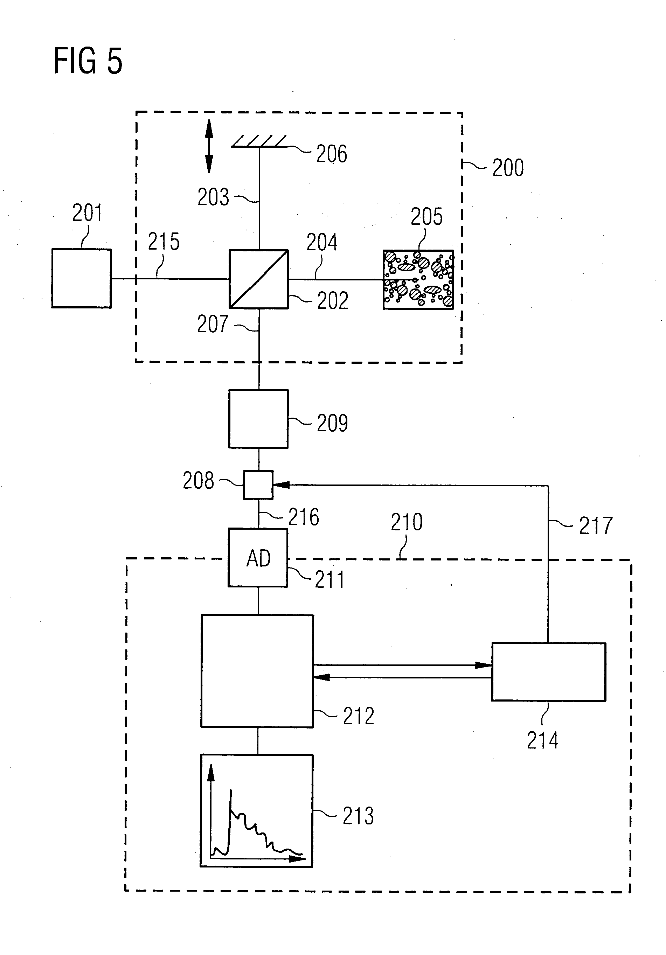

Bei der bevorzugten Ausführung von **Fig. 2** befindet sich im Referenzarm 203 eine adaptive analoge Signalverarbeitungseinheit 208. Diese fungiert insbesondere als variables optisches Dämpfungsglied. Da das Referenzlicht der Verstärkung des Messsignals dient, führt eine optische Dämpfung im Referenzarm zu einer Abschwächung des analogen Interferenzsignals, das dem A/D-Wandler 211 zugeführt wird. In entsprechender Weise führt eine Verstärkung im Referenzarm zu einer Verstärkung des analogen Interferenzsignals und damit auch zu einer Verstärkung des elektrischen Signals 216. Bei der in **Fig. 3** dargestellten bevorzugten Ausführungsform befindet sich eine adaptive analoge Signalverarbeitungseinheit 208 im Probenarm 204 und fungiert als variables optisches Dämpfungsglied, das direkt auf die von der Probe 205 zurückgestreute optische Leistung wirkt. Bei der in **Fig. 4** dargestellten bevorzugten Ausführungsform ist ein variables optisches Dämpfungsglied als adaptive analoge Signalverarbeitungseinheit 208 im Detektionsarm 207 vor dem Detektor 209 angeordnet. In diesem Fall werden Signal- und Referenzlicht gleichermaßen gedämpft oder verstärkt. In der in **Fig. 5** dargestellten bevorzugten Ausführungsform befindet sich die adaptive analoge Signalverarbeitungseinheit 208 zwischen Detektor 209 und dem Analog/Digital-Umsetzer (A/D-Umsetzer) 211 und fungiert als variables elektrisches Verstärkungs- und/oder Dämpfungsglied.

Die vorstehend beschriebenen Anordnungen lassen sich in analoger Weise auch auf andere OCT-Systeme übertragen. Dazu zählen beispielsweise Konfigurationen auf Basis von Mach-Zehnder-Interferometern oder Systeme mit differentieller optischer Detektion. Ebenso sind Kombinationen der in den Figuren 2 bis 5 skizzierten Anordnungen möglich, bei denen eine tiefenabhängige Gewichtung des analogen Messsignals durch Eingriffe an mehreren Stellen des optischen und/oder elektrischen Signalpfades erreicht wird.

Die analoge Signalverarbeitungseinheit 208 ändert ihre Dämpfungseigenschaften vorzugsweise mehrmals während eines Tiefenscans (A-Scans). Dies kann kontinuierlich und/oder zu diskreten Zeiten erfolgen. Um eine ausreichende Anpassbarkeit zu erreichen, wird im Falle einer zeitlich diskreten Anpassung die optische Dämpfung während eines Tiefenscans bevorzugt mindestens 5 mal, besonders bevorzugt mindestens 20 mal verändert.

Die in der Anordnung nach Fig. 5 verwendete analoge elektrische Signalverarbeitungseinheit 208 wird technisch vorzugsweise unter Verwendung eines variablen rauscharmen Verstärkers und/oder unter Verwendung eines rauscharmen Verstärkers mit nachgeschaltetem regelbarem Dämpfungsglied realisiert. Der Verstärker ist vorzugsweise ein CMOS-Verstärker oder besonders bevorzugt ein GaAs-HEMT-Verstärker. Das variable Dämpfungsglied ist vorzugsweise als digitales Stufendämpfungsglied vorgeschlagen, um die Steuerung von einem Messrechner aus zu vereinfachen.

Um die analoge Signalverarbeitungseinheit adaptiv zu steuern umfasst die Datenerfassungs- und -verarbeitungseinheit 210 vorzugsweise ein Steuermodul 214, welches mit dem Datenauswertungsmodul 212 zur Überprüfung des Erreichens von Dynamik-Grenzen des A/D-Umsetzers 211 in Signalverbindung steht. Das Datenauswertungsmodul 212 ist also insbesondere ausgelegt, zu untersuchen, ob das quantisierte Messsignal den dynamischen Bereich des Umsetzers 211 über- oder unterschreitet, ob also die obere oder die untere Grenze des Dynamikbereichs überschritten wird. Ist dies der Fall, werden Steuerwerte, insbesondere Werte, welche die Messtiefe festlegen, in der die Bereichsverletzung auftritt, an das Steuermodul 214 übermittelt, welches einerseits über ein Steuersignal 217 die analoge Signalverarbeitungseinheit 208 nachregelt, andererseits das Datenauswertungsmodul 212 derart steuert, dass das digitalisierte Signals des sich ergebenen A-Scans derart korrigiert wird, dass der Einfluss der analogen Signalverarbeitungseinheit 208 im digitalisierten Signal kompensiert wird, bevor das Signal zur Darstellung 213 kommt. Die Überprüfung von Bereichsüberschreitungen der Dynamik des A/D-Umsetzers 211 mittels des Datenauswertungsmoduls 212 und eine eventuelle Nachregelung der Wirkung der analogen Signalverarbeitungseinheit 208 mittels des Steuermoduls 214 über das Steuersignal 217 erfolgt somit insbesondere in Form eines adaptiven, geschlossenen Regelkreises.

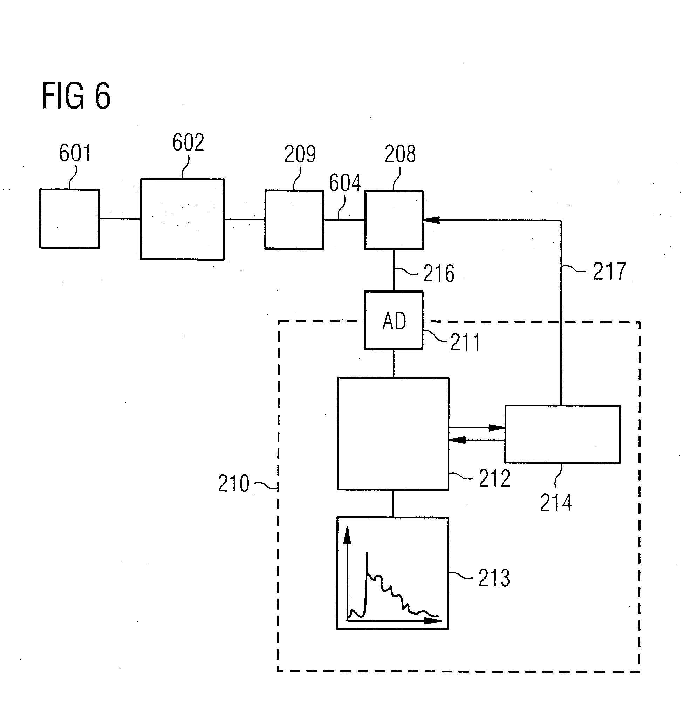

Eine tiefenselektive Gewichtung des Messsignals im SS-OCT wird vorzugsweise durch eine Filterung des analog-elektrischen Signals mit einem linearen zeitinvarianten Filter erreicht. Eine entsprechende Anordnung gemäß einer bevorzugten Ausführungsform ist in **Fig. 6** gezeigt. Soweit dieselben Bezugszeichen verwendet sind wie in den vorangegangen beschriebenen Ausführungsformen, lassen sich die Ausführungen dazu vorzugsweise in analoger Weise auf die Ausführungsform von Fig. 6 anwenden.

Licht eines Swept-Source-Lasers 601 wird in ein Messinterferometer 602 eingekoppelt. Dieses ist beispielsweise als Michelson- oder Mach-Zehnder-Interferometer ausgeführt und beinhaltet vorzugsweise einen Referenzarm, einen Probenarm sowie eine zu untersuchende Probe. Die sich überlagernden Lichtwellen aus dem Referenz- und dem Probenarm treten in Interferenz und werden in einem Fotodetektor 209 quadratisch empfangen. Das entstehende analog-elektrische Messsignal 604 besteht aus niederfrequenten Anteilen aus kleineren Pfadlängenunterschieden, die von der Streuung in kleineren Tiefen herrühren, und aus höherfrequenten Anteilen aus größeren Probentiefen. Diese Signalanteile werden durch eine frequenzselektive Filterung in einer adaptiven elektrisch-analogen Signalverarbeitungseinheit 208 nivelliert.

Diese Signalverarbeitungseinheit 208 ist beispielsweise als Kombination eines linearen zeitinvarianten Filters mit einem variablen Verstärkerglied ausgeführt. Das Filter kann wiederum mehrere kaskadierte Stufen enthalten. Bei diesen Stufen kann es sich beispielsweise um folgende Systeme handeln:
- Hochpassfilter mit einer Eckfrequenz, die vorzugsweise der doppelten bis zehnfachen Rate entspricht, mit der A-Scans aufgenommen werden;
- Ein abstimmbares Kerbfilter hoher Güte, das schmalbandige Beiträge von starken Streuereignissen (z.B. Oberflächenreflexionen der Probe) unterdrückt.
- Ein Hochpassfilter mit einer exponentiell ansteigenden Transmissionscharakteristik, das Leistungsunterschiede aufgrund optischer Dämpfung innerhalb der Probe kompensiert.

Die Hochpassfilter können beispielsweise als passive LC- oder aktive ButterworthFilter erster oder zweiter Ordnung ausgeführt werden - dies gewährleistet einen flachen Phasengang im Durchlassbereich. Die Filter weisen damit eine Flankensteilheit von vorzugsweise 20 dB bzw. 40 dB je Dekade auf. Das Kerbfilter wird vorzugsweise passiv als RC-twin-T realisiert und vorzugsweise in einer Sallenand-Key-Konfiguration mit aktiven Komponenten verschaltet. Weiter bevorzugt kommt hier die Verwendung eines aktiven Filterbausteins in Betracht.

In einer weiteren bevorzugten Ausführungsform ist das spektrale Filter als Filterbank aufgebaut. Diese kann aus parallelgeschalteten Kombinationen aus Bandpässen und Dämpfungsgliedern bestehen. Dadurch lassen sich einzelne Frequenzbänder gezielt ansteuern. Die Einzelbandpässe können wiederum als passive LC-Filter oder als aktive Bausteine zur Verwendung kommen. Das variable Verstärkungsglied kann technisch unter Verwendung eines variablen rauscharmen Verstärkers oder unter Verwendung eines rauscharmen Verstärkers mit nachgeschaltetem regelbarem Dämpfungsglied realisiert werden. Der Verstärker ist vorzugsweise ein CMOS-Verstärker oder besonders bevorzugter Weise ein GaAs-HEMT-Verstärker. Das variable Dämpfungsglied wird als digitales Stufendämpfungsglied vorgeschlagen, um die Steuerung von einem Messrechner aus insbesondere mittels des Steuermoduls 214 der Datenerfassungs- und -verarbeitungseinheit 210 zu vereinfachen.

Das aufbereitete analoge Signal 216 wird dann im Analog/Digital-Umsetzer 211 abgetastet und quantisiert. Dieser Umsetzer besitzt bevorzugt eine Quantisierungstiefe im Bereich von etwa 8 bit bis etwa 16 bit, besonders bevorzugt im Bereich von etwa 12 bit bis etwa 16 bit. In der Datenerfassungs- und -verarbeitungseinheit 210 wird das digitale Signal nun vorzugsweise mehreren digitalen Aufbereitungsschritten 212 unterzogen, bei dem der Einfluss der analog-elektrischen Signalverarbeitungseinheit 208 rückgängig gemacht wird, bevor das Signal zur Darstellung 213 kommt. Wie bereits oben ausgeführt, wird im digitalen Aufbereitungsschritt 212 insbesondere untersucht, ob das quantisierte Messsignal den dynamischen Bereich des Umsetzers 211 über- oder unterschreitet. Ist dies der Fall, werden entsprechende Informationen an das Steuermodul 214 übermittelt, welches sowohl die analog-elektrische Signalverarbeitungseinheit 208 als auch die digitale Korrektur im Datenauswertungsmodul 212 nachregelt und insbesondere die Aufnahme eines neuen A-Scans an diesem Punkt auslöst.

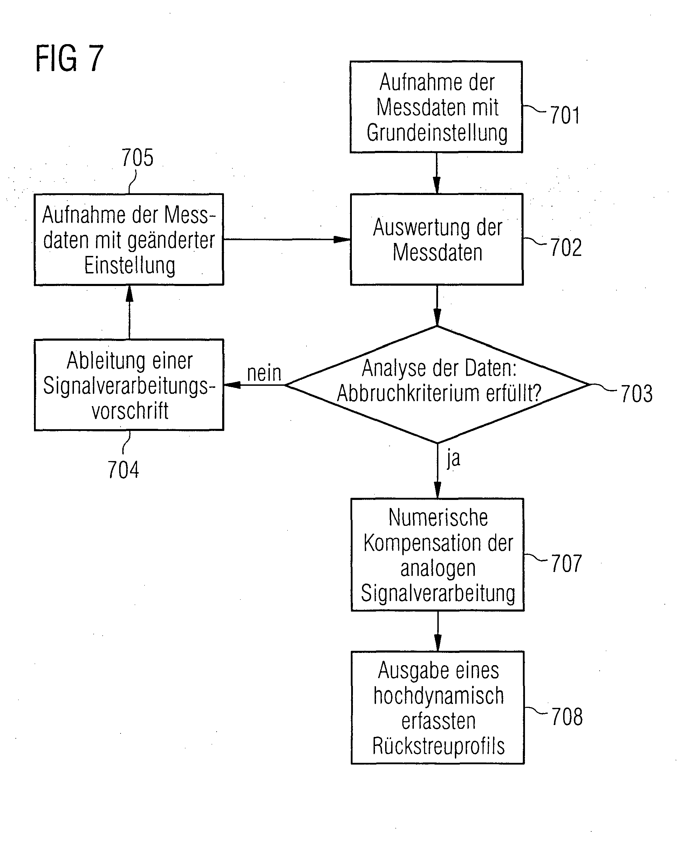

Fig. 7 zeigt ein Flussdiagramm zur Veranschaulichung eines Verfahren gemäß einer bevorzugten Ausführungsform der vorliegenden Erfindung. Damit wird insbesondere angewendet auf ein OCT-System mit analoger Signalverarbeitungseinheit (z.B. nach Fig. 2-6), allgemein die Entkopplung des Gesamtdynamikbereichs einer Tiefenrückstreumessung (A-Scan) vom Dynamikbereich des A/D-Wandlers 211 bewirkt, und somit eine Erweiterung des Dynamikbereichs der Messung ermöglicht.

Das dargestellte Verfahren umfasst insbesondere die folgenden Schritte:
701. Aufnahme eines A-Scans mit einer ersten Einstellung der analogen Signalverarbeitungseinheit. Die Aufnahme beinhaltet somit vorzugsweise einen Tiefenscan des Referenzreflektors bei TD-OCT bzw. einen Frequenzscan des Swept-Source-Lasers bei SS-OCT, sowie eine Umsetzung in ein analog-elektrisches Messsignal und dessen Analog/Digital-Wandlung.
702. Digitale Signalverarbeitung der Messdaten und Ermittlung des tiefenabhängigen Intensitätsprofils.
703. Untersuchung des tiefenabhängigen Intensitätsprofils auf Verletzung des Dynamikbereichs des Analog/Digital-Umsetzers.
704. Wurde der Dynamikbereich lokal verletzt, beispielsweise durch Überschreiten des Spannungsmessbereiches oder durch Unterschreiten der kleinsten Quantisierungsstufe, wird eine zweite Einstellung der analogen Signalverarbeitungseinheit ermittelt, die den Bereichsverletzungen entgegenwirkt.
705. Wiederholte Aufnahme eines weiteren A-Scans unter Einfluss der geänderten Parameter für die analoge Signalverarbeitungseinheit.
706. Iterative Wiederholung der Schritte 702-705, bis in Schritt 703 ein Abbruchkriterium erfüllt wird. Dieses kann eine Nichtverletzung des Dynamikbereichs des Umsetzers sein oder das Erreichen technischer Grenzen der Systemkomponenten beinhalten.
707. Numerische (digitale) Kompensation des Einflusses der adaptiven analogen Signalverarbeitung auf die Messdaten.
708. Ausgabe / Speicherung / Auswertung des hochdynamisch erfassten ortsabhängigen Rückstreuprofils.

Nachfolgend werden der Vollständigkeit halber jeweils ein Ausführungsbeispiel für ein SS-OCT und ein TD-OCT beschrieben.

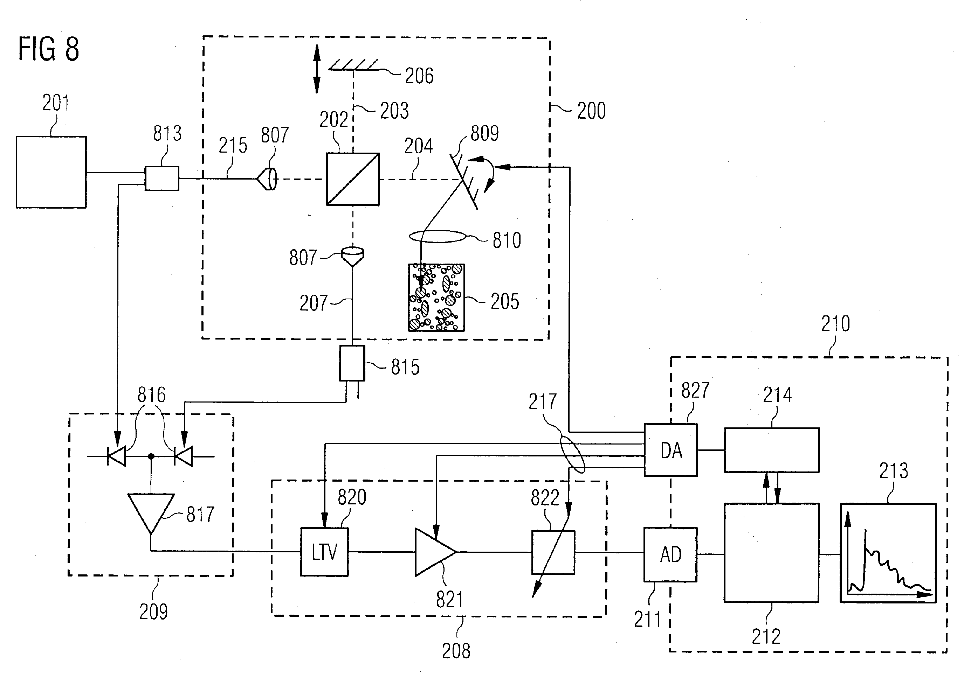

Ein bevorzugtes Ausführungsbeispiel zur Implementierung der vorliegenden Erfindung wird im Folgenden anhand eines hochdynamischen TD-OCT-System mit Bezug auf **Fig.8** beschrieben, bei dem eine analog-elektrische Signalverarbeitungseinheit 208 zwischen einem Detektor 209 und einem A/D-Wandler 211 angeordnet ist. Das Beispiel beruht auf einem Michelson-Interferometer 200 als Messinterferometer. Dieses umfasst einen halbdurchlässigen Spiegel 202 (als Strahlteiler), einen Beleuchtungsarm 215, einen Probenarm 204, einen Referenzarm 203 und einen Detektionsarm 207. Das Interferometer ist vorzugsweise freistrahl-optisch aufgebaut. Zwei Faserkollimatoren 807 dienen der Ein- und Auskopplung des Lichtstrahls. Eine Superlumineszenzdiode als Weißlichtquelle 201, mit einer spektralen Bandbreite von 110 nm und einer Mittenwellenlänge von 1330 nm befindet sich im Beleuchtungsarm 215.

Der Probenarm 204 umfasst vorzugsweise eine Strahlablenkungseinheit 809, bestehend aus zwei Galvo-Scan-Spiegeln, je einen für jede transversale Ablenkungsrichtung. Im Probenarm 204 folgt eine Scan-Linse 810, welche auf die Strahlablenkungseinheit abgestimmt ist und den dort erzeugten Ablenkungswinkel in einen transversalen Versatz umwandelt. Ebenso dient die Scan-Linse 810 der Fokussierung des kollimierten Lichtstrahls in die Probe 205. Der Referenzarm 203 umfasst vorzugsweise einen verschiebbaren Referenzreflektor 206.

In diesem Ausführungsbeispiel wird ein differentielles Empfangsschema angewendet. Daher erfüllt der Beleuchtungsarm 215 zusätzlich die Aufgabe eines zweiten Detektionsarmes. Die optische Leistung wird dafür mittels eines ersten 50:50-Kopplers 813 teilweise aus dem Beleuchtungsarm 215 ausgekoppelt und wie das Licht aus dem Detektionsarm 207 dem differentiellen Detektor 209 zugeführt. Um gleiche Signalintensitäten an beiden Detektoreingängen zu erreichen, befindet sich ein zweiter 50:50-Koppler 815 im Detektionsarm. Der differentielle Empfänger ist insbesondere aus zwei in Reihe geschalteten Fotodioden 816 und einem Transimpedanzverstärker 817 aufgebaut. Die optische Leistungsdifferenz beider Eingänge wird proportional in ein Spannungssignal am Ausgang umgesetzt.

Die analoge Signalverarbeitungseinheit 208 befindet sich zwischen Detektor und dem Analog/Digital-Umsetzer 211, welcher beispielsweise als 16 bit-A/D-Umsetzer ausgebildet ist. Sie umfasst im Einzelnen insbesondere ein lineares zeitvariantes Element (LTV) 820, einen rauscharmen Verstärker 821 und ein digitales Stufendämpfungsglied 822. Dabei ist das lineare zeitvariante Element in dieser Ausführung vorzugsweise ein variabler elektrischer Attenuator. Die Datenerfassungs- und -verarbeitungseinheit 210 beinhaltet vorzugsweise ein numerisches Datenauswertungsmodul 212, ein Steuermodul 214 und eine Datenausgabeeinheit 213. Das numerische Datenauswertungsmodul dient der Verarbeitung des digitalisierten Signals. Dies beinhaltet insbesondere die Rekonstruktion des Rückstreuprofils aus den Messdaten, die numerische Korrektur des Einflusses der analogen Signalverarbeitung und die Erkennung eventueller lokaler Überschreitungen des Dynamikbereiches des Analog/Digital-Umsetzers 211. Solche Bereichsüberschreitungen können beispielsweise durch mehrere, aufeinander folgende Werte am oberen oder unteren Rand des Dynamikbereichs des A/D-Umsetzers erkannt werden.

Das Steuermodul 214 hat insbesondere zur Aufgabe, diesen Bereichsüberschreitungen durch Ansteuerung der analogen Signalverarbeitungseinheit 208 entgegenzuwirken, indem das analoge Messsignal an der Stelle der lokalen Bereichsüber- bzw. -unterschreitung abgeschwächt bzw. verstärkt wird. Ebenso steuert das Steuermodul 214 den numerischen Korrekturschritt, also die digitale Kompensation des Einflusses der analogen Signalverarbeitungseinheit 208 mittels des Datenauswertungsmoduls 212, und die Strahlablenkungseinheit im Probenarm 204. Steuersignale des Steuermoduls 214, die die Datenerfassungs- und -verarbeitungseinheit 201 verlassen, werden dafür vorzugsweise mittels eines Digital/Analog-Wandler (D/A) 827 umgesetzt.

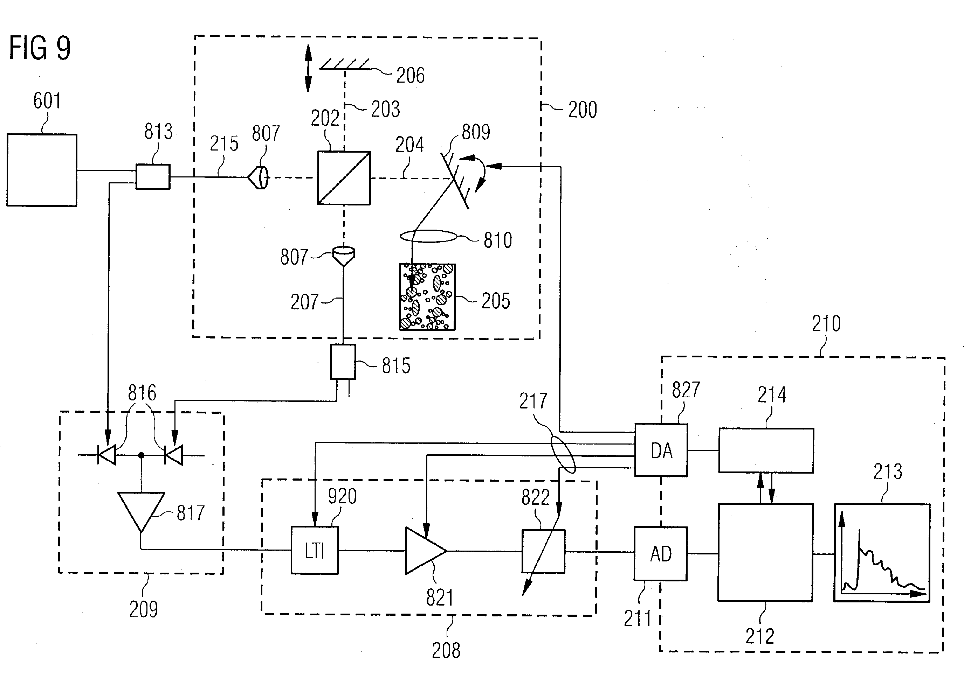

Eine mögliche Implementierung der Erfindung in einem SS-OCT-System mit erweitertem Dynamikbereich gemäß einer weiteren bevorzugten Ausführungsform ist in Fig. 9 gezeigt. Dort ist ein Michelson-Interferometer 200 als Messinterferometer gezeigt. Dieses umfasst einen halbdurchlässigen Spiegel 202 als Strahlteiler, einen Beleuchtungsarm 215, einen Probenarm 204, einen Referenzarm 203 und einen Detektionsarm 207. Das Interferometer ist vorzugsweise freistrahl-optisch aufgebaut. Zwei Faserkollimatoren 807 dienen der Ein- und Auskopplung des Lichtstrahls. Eine Swept-Source 601, abstimmbar über den Wellenlängenbereich von 1240 nm bis 1380 nm, mit einer Scanrate von beispielsweise 20 kHz befindet sich im Beleuchtungsarm 215. Der Probenarm 204 enthält eine Strahlablenkungseinheit 809, welche insbesondere zwei Galvo-Scan-Spiegel umfasst, je einen für jede transversale Ablenkungsrichtung. Im Probenarm 204 folgt eine Scan-Linse 810, welche auf die Strahlablenkungseinheit 809 abgestimmt ist und den dort erzeugten Ablenkungswinkel in einen transversalen Versatz umwandelt. Ebenso dient die Scan-Linse 810 der Fokussierung des kollimierten Lichtstrahls in die folgende Probe 205. Der Referenzarm enthält einen fest positionierten Referenzreflektor 206.

Auch in diesem Ausführungsbeispiel wird ein differentielles Empfangsschema angewendet. Daher erfüllt der Beleuchtungsarm 215 zusätzlich die Aufgabe eines zweiten Detektionsarmes. Die optische Leistung wird dafür mittels eines ersten 50:50-Kopplers 813 teilweise aus dem Beleuchtungsarm 215 ausgekoppelt und wie das Licht aus dem Detektionsarm dem differentiellen Detektor 209 zugeführt. Um gleiche Signalintensitäten an beiden Detektoreingängen zu erreichen, befindet sich ein zweiter 50:50-Koppler 815 im Detektionsarm 207. Der differentielle Empfänger 209 ist aus zwei in Reihe geschalteten Photodioden 816 und einem Transimpedanzverstärker 817 aufgebaut. Die optische Leistungsdifferenz beider Eingänge wird proportional in ein Spannungssignal am Ausgang umgesetzt.

Die analoge Signalverarbeitungseinheit 208 befindet sich zwischen Detektor 209 und dem Analog/Digital-Umsetzer 211, welcher beispielsweise als 12-bit-Umsetzer ausgestaltet ist. Sie umfasst im Einzelnen insbesondere eine adaptive Filtereinheit (LTI) 920, einen rauscharmen Verstärker 821 und ein digitales Stufendämpfungsglied 822. Dabei ist die adaptive Filtereinheit vorzugsweise als Reihenschaltung eines LC-Butterworth-Hochpassfilters zweiter Ordnung, mit Grenzfrequenz 200 kHz, sowie eines Kerbfilters mit variabler Sperrfrequenz, sowie eines aktiven Hochpassfilters in Butterworth-Konfiguration mit einstellbarer Grenzfrequenz im Bereich von etwa 2 MHz bis etwa 20 MHz aufgebaut.

Die Datenerfassungs- und -verarbeitungseinheit 210 umfasst insbesondere ein numerisches Datenauswertungsmodul 212, ein Steuermodul 214 und eine Datenausgabeeinheit 213. Das numerische Datenauswertungsmodul ist insbesondere zur Fourier-Transformation und Rückrechnung des gemessenen Signals auf Rückstreutiefen, einschließlich numerischer Korrektur der analogen Signalverarbeitung und Erkennung eventueller lokaler Überschreitungen des Dynamikbereiches des Analog/Digital-Umsetzers 211 ausgelegt. Solche Bereichsüberschreitungen können beispielsweise durch mehrere, aufeinander folgende nicht-Fourier-transformierte Werte am oberen Rand des Dynamikbereichs des A/D-Umsetzers oder durch mehrere aufeinander folgende Fourier-transformierte Werte an der unteren Rauschgrenze des Systems erkannt werden. Diese Rauschgrenze kann an Stellen des Tiefenscans abgelesen werden, an denen keine Rückstreuung erwartet wird, beispielsweise an Luft.

Das Steuermodul 214 ist ausgelegt, diesen Bereichsüberschreitungen durch Ansteuerung der analogen Signalverarbeitungseinheit 208 entgegenzuwirken. Dazu wird die spektrale Filtercharakteristik angepasst und die Verstärkungs- und Dämpfungseigenschaften des Verstärkungs- und Dämpfungsgliedes angepasst. Beispielsweise wird das Frequenzband, in welchem die Facettenreflexion erscheint gezielt abgedämpft. Dieser Anpassungsprozess geschieht vorzugsweise iterativ. Besonders bevorzugt wird zunächst das gesamte Reflektivitätsprofil stark genug abgedämpft, dass keine Überschreitung des Dynamikbereichs des A/D-Umsetzers 211 nach oben auftritt. Dies wird vor der Fourier-Transformation überprüft. Im nächsten Schritt findet vorzugsweise eine Fourier-Transformation des Signals statt. Das danach vorliegende Signal zeigt, welche Frequenzanteile mit größerer Leistung auftreten. Daraufhin kann das lineare zeitinvariante Filter 920 so eingestellt werden, dass die stärkeren Frequenzanteile abgeschwächt werden. Im Gegenzug wird die Gesamtleistung des Eingangssignals wieder angehoben, bzw. die Dämpfung reduziert. Treten weiterhin Überschreitungen des Dynamikbereichs des A/D-Umsetzers auf, werden die Anpassungsschritte wiederholt.

Vorzugsweise ist das Steuermodul 214 außerdem ausgelegt, die numerische Kompensation der Wirkung der analogen Signalverarbeitungseinheit 209 im Datenauswertungsmodul 212 und die Strahlablenkungseinheit 809 im Probenarm 204 zu steuern. Steuersignale, welche die Datenerfassungs- und -verarbeitungseinheit 210 verlassen, werden dafür in einem Digital/Analog-Wandler (DA) 827 umgesetzt.

### Bezugszeichenliste

- 110: Rückstreuprofil
- 111: Anstieg
- 112: Signalspitze
- 113: exponentiell abfallender Verlauf der Rückstreuung
- 114: zu untersuchende Probe
- 115: herkömmliche Signaluntergrenze
- 116: Untergrenze des Messbereichs
- 117: prinzipielle (analoge) Empfindlichkeitsgrenze
- 118: Pegel des Quantisierungsrauschens des A/D-Wandlers
- 119: Messbereich
- 120: Dynamikbereich des A/D-Wandlers
- 121: Hochpassbereich
- 122: Kerbfilter
- 123: exponentiell mit Frequenz ansteigende Hochpasscharakteristik
- 124: Gewichtungsvorschrift
- 125: effektive Dynamik des Systems
- 131: modifiziertes Rückstreuprofil
- 140: rekonstruiertes Rückstreuprofil
- 141: tiefenabhängiger Messbereich
- 200: Michelson-Interferometer
- 201: breitbandige Lichtquelle
- 202: Strahlteiler
- 203: Referenzarm
- 204: Probenarm
- 205: Probe
- 206: Referenzreflektor
- 207: Detektionsarm
- 208: adaptive analoge Signalverarbeitungseinheit
- 209: Detektor
- 210: Datenerfassungs- und -verabeitungseinheit
- 211: Analog/Digital-Umsetzer
- 212: Datenauswertungsmodul
- 213: Datenausgabeeinheit
- 214: Steuermodul
- 215: Beleuchtungsarm
- 216: analoges Messsignal
- 217: Steuersignal
- 601: abstimmbare Laserquelle
- 602: Messinterferometer
- 604: Messsingal
- 807: Faserkollimator
- 809: Strahlablenkungseinheit
- 810: Scan-Linse
- 813: optischer Koppler
- 815: optischer Koppler
- 816: Photodiode
- 817: Transimpedanzverstärker
- 820: lineares zeitvariantes Element
- 821: elektrischer Verstärker
- 822: digitales Stufendämpfungsglied
- 827: Digital/Analog-Umsetzer
- 920: lineares zeitinvariantes Element

## Patentansprüche

1. System zur optischen Kohärenztomographie, umfassend:
- eine optische Detektionseinheit (200, 201, 209) zur optischen Erfassung eines messtiefenabhängigen optischen Rückstreuprofils (110), wobei die optische Detektionseinheit (200, 201, 209; 601, 602, 209) ausgelegt ist, ein analoges Messsignal (216) des erfassten messtiefenabhängigen optischen Rückstreuprofils (110) zu generieren, wobei die optische Detektionseinheit eine steuerbare analoge Signalverarbeitungseinheit (208) umfasst, welche ausgelegt ist, in Abhängigkeit von einem erfassten Steuersignal (217) eine selektive Gewichtung der zu verschiedenen Messtiefen gehörenden Anteile des analogen Messsignals vorzunehmen;
- eine Signalwandlereinheit (211), welche ausgelegt ist, das analoge Messsignal (216) der optischen Detektionseinheit zu erfassen und in ein digitales Signal zu wandeln; und
- eine Datenerfassungs- und -verarbeitungseinheit (210), welche ausgelegt ist, Über- und/oder Unterschreitungen eines Dynamikbereichs der Signalwandlereinheit (211) zu ermitteln, die analoge Signalverarbeitungseinheit mittels des Steuersignals derart zu steuern, dass einer ermittelten Über- und/oder Unterschreitung des Dynamikbereichs der Signalwandlereinheit entgegengewirkt wird und eine Korrektur des digitalen Signals derart vorzunehmen, dass dadurch die von der Signalverarbeitungseinheit vorgenommene Gewichtung verschiedener Signalanteile kompensiert wird.

2. System nach Anspruch 1, wobei die optische Detektionseinheit umfasst:
- eine Lichtquelle (601), deren Wellenlänge innerhalb eines Scan-Bereichs der optischen Detektionseinheit zeitlich veränderbar ist; und
- einen optischen Detektor (209), der ausgelegt ist, ein optisches Signal der optischen Detektionseinheit in ein analoges elektrisches Messsignal (604) zu wandeln,
wobei die analoge Signalverarbeitungseinheit (208) ein adaptives lineares zeitinvariantes Filter umfasst, welches eine Filterung des elektrischen Messsignals vornimmt.

3. System nach Anspruch 2, wobei das adaptive Filter einen Hochpassfilter und/oder ein Bandpassfilter und/oder einen Kerbfilter und/oder eine breitbandige Verstärkungseinheit umfasst.

4. System nach Anspruch 1, wobei die optische Detektionseinheit ein optisches Interferometer (200) mit einem Probenarm (204) und einem Referenzarm (203) umfasst, wobei ein optischer Gangunterschied zwischen dem Probenarm (204) und dem Referenzarm (203) zur Abtastung der Messtiefe zeitlich veränderbar ist, und wobei die analoge Signalverarbeitungseinheit (208) ein zeitlich variables lineares Verstärkungs- und/oder Dämpfungsglied umfasst, das von der Datenerfassungs- und -verarbeitungseinheit (210) in Abhängigkeit vom optischen Gangunterschied zwischen dem Probenarm (204) und dem Referenzarm (203) gesteuert wird.

5. System nach Anspruch 4, wobei das Verstärkungs- und/oder Dämpfungsglied ein elektrisches Verstärkungs- und/oder Dämpfungsglied umfasst.

6. System nach Anspruch 4, wobei das Verstärkungs- und/oder Dämpfungsglied ein optisches Verstärkungs- und/oder Dämpfungsglied umfasst.

7. Verfahren zur optischen Kohärenztomographie umfassend:
- Erfassen eines messtiefenabhänigen optischen Rückstreuprofils mittels einer optischen Detektionseinheit, die ein analoges Messsignal erzeugt;
- Wandeln des analogen Messsignals in ein digitales Signal mittels einer Signalwandlereinheit (211);
- Ermitteln einer Über- und/oder Unterschreitung eines Dynamikbereichs der Signalwandlereinheit (211);
- selektives Gewichten der zu verschiedenen Messtiefen gehörenden Anteile des analogen Messsignals mittels einer adaptiven analogen Signalverarbeitungseinheit (208) derart, dass einer ermittelten Über- und/oder Unterschreitung des Dynamikbereichs entgegengewirkt wird;
- Korrigieren des digitalen Signals derart, dass dadurch die mittels der analogen Signalverarbeitungseinheit vorgenommene Gewichtung verschiedener Anteile des analogen Signals kompensiert wird.

8. Verfahren nach Anspruch 7, wobei das Erfassen des optischen Rückstreuprofils, das Generieren eines analogen Messsignals und das Ermitteln einer Über- und/oder Unterschreitung des Dynamikbereichs zunächst für eine erste Einstellung der adaptiven analogen Signalverarbeitungseinheit durchgeführt wird, wobei anschließend in Abhängigkeit von der erfassten Über- und/oder Unterschreitung des Dynamikbereichs eine angepasste Einstellung der adaptiven analogen Signalverarbeitungseinheit vorgenommen wird, und wobei das Erfassen des optischen Rückstreuprofils, das Generieren eines analogen Messsignals und das Ermitteln einer Über- und/oder Unterschreitung des Dynamikbereichs bei einer jeweils angepassten Einstellung der analogen Signalverarbeitungseinheit solange wiederholt werden, bis ein vorgegebenes Abbruchkriterium erfüllt wird, und wobei das Korrigieren des digitalen Signals gemäß der letzten Einstellung der analogen Signalverarbeitungseinheit erfolgt.

9. Computerimplementiertes Verfahren zur Steuerung einer optischen Kohärenztomographie-Messung, umfassend
- Erfassen eines von einer Signalwandlereinheit erzeugten digitalen Signals eines messtiefenabhängigen Rückstreuprofils einer optischen Kohärenztomographie-Messung;
- Ermitteln einer Über- und/oder Unterschreitung eines Dynamikbereichs der Signalwandlereinheit;
- Generieren eines Steuersignals zur Steuerung einer adaptiven analogen Signalverarbeitungseinheit derart, dass eine durch das Steuersignal von der adaptiven analogen Signalverarbeitungseinheit bewirkte selektive Gewichtung von zu verschiedenen Messtiefen gehörenden analogen Signalanteile des Rückstreuprofils der optischen Kohärenztomographie-Messung der ermittelten Über- und/oder Unterschreitung des Dynamikbereichs entgegenwirkt; und
- Korrigieren des digitalen Signals derart, dass dadurch die von der Signalverarbeitungseinheit vorgenommene selektive Gewichtung verschiedener Signalanteile kompensiert wird.

10. Computersystem zur Steuerung einer optischen Kohärenztomographie-Messung, umfassend
- einen digitalen Signaleingang zum Erfassen eines von einer Signalwandlereinheit erzeugten digitalen Signals eines messtiefenabhängigen Rückstreuprofils einer optischen Kohärenztomographie-Messung;
- ein Datenauswertungsmodul zum Ermitteln einer Über- und/oder Unterschreitung eines Dynamikbereichs der Signalwandlereinheit;
- einen Datenspeicher mit einer Steuervorschrift, welche für Über- und/oder Unterschreitungen des Dynamikbereichs der Signalwandlereinheit Werte eines Steuersignals zur Steuerung einer adaptiven analogen Signalverarbeitungseinheit derart festlegt, dass eine durch das Steuersignal von der adaptiven analogen Signalverarbeitungseinheit bewirkte selektive Gewichtung von zu verschiedenen Messtiefen gehörenden analogen Signalanteile des Rückstreuprofils der optischen Kohärenztomographie-Messung den Über- und/oder Unterschreitungen des Dynamikbereichs entgegenwirkt; und
- ein Steuermodul, welches der ermittelten Über- und/oder Unterschreitung des Dynamikbereichs gemäß der im Datenspeicher hinterlegten Steuervorschrift ein Steuersignal generiert,
wobei das Datenauswertungsmodul ausgelegt ist, das digitale Signal derart zu korrigieren, dass dadurch die gemäß der Steuervorschrift für das erzeugte Steuersignal von der Signalverarbeitungseinheit bewirkte selektive Gewichtung verschiedener Signalanteile kompensiert wird.

## Claims

1. A system for optical coherence tomography, comprising:
- an optical detection unit (200, 201, 209) for optically acquiring a measurement depth-dependent optical backscattering profile (110), wherein the optical detection unit (200, 201, 209; 601, 602, 209) is designed to generate an analog measurement signal (216) of the acquired measurement depth-dependent optical backscattering profile (110), wherein the optical detection unit comprises a controllable analog signal processing unit (208), which is designed to perform selective weighting of the portions of the analog measurement signal belonging to different measurement depths, depending on an acquired control signal (217);
- a signal converter unit (211), which is designed to acquire the analog measurement signal (216) of the optical detection unit, and to convert the signal into a digital signal; and
- a data acquisition and processing unit (210), which is to detect overshoots and/or undershoots of a dynamic range of the signal converter unit (211), to control the analog signal processing unit by means of the control signal such that a detected overshoot and/or undershoot of the dynamic range of the signal converter unit is counteracted, and to perform a correction of the digital signal such that thereby the weighting of different signal portions performed by the signal processing unit, is compensated.

2. The system according to claim 1, wherein the optical detection unit comprises:
- a light source (601), the wavelength of which can be changed over time within a scan range of the optical detection unit; and
- an optical detector (209), which is designed to convert an optical signal of the optical detection unit into an analog electrical measurement signal (604),
wherein the analog signal processing unit (208) comprises an adaptive linear time invariant filter, which performs a filtering of the electrical measurement signal.

3. The system according to claim 2, wherein the adaptive filter comprises a high-pass filter and/or a band-pass filter and/or a notch filter and/or a broadband amplification unit.

4. The system according to claim 1, wherein the optical detection unit comprises an optical interferometer (200) having a sample arm (204) and a reference arm (203), wherein an optical path difference between the sample arm (204) and the reference arm (203) can be changed over time to the scanning of the measurement depth, and wherein the analog signal processing unit (208) comprises a linear amplifier and/or attenuator that is variable over time, that is controlled by the data acquisition and processing unit (210) depending on the optical path difference between the sample arm (204) and the reference arm (203).

5. The system according to claim 4, wherein the amplifier and/or attenuator comprises an electrical amplifier and/or attenuator.

6. The system according to claim 4, wherein the amplifier and/or attenuator comprises an optical amplifier and/or attenuator.

7. A method for optical coherence tomography comprising:
- acquiring a measurement depth-dependent optical backscattering profile by means of an optical detection unit, which generates an analog measurement signal;
- converting the analog measurement signal into a digital signal by means of a signal converter unit (211);
- detecting an overshoot and/or undershoot of a dynamic range of the signal converter unit (211);
- selective weighting of the portions of the analog measurement signal belonging to different measurement depths by means of an adaptive analog signal processing unit (208) such that a detected overshoot and/or undershoot of the dynamic range is counteracted;
- correcting the digital signal in such a manner that thereby the weighting of different portions of the analog signal performed by means of the analog signal processing unit, is compensated.

8. The method according to claim 7, wherein acquiring the optical backscattering profile, generating an analog measurement signal and detecting an overshoot and/or undershoot of the dynamic range is initially performed for a first setting of the adaptive analog signal processing unit, wherein next, an adjusted setting of the adaptive analog signal processing unit is performed depending on the acquired overshoot and/or undershoot of the dynamic range, and wherein acquiring the optical backscattering profile, generating an analog measurement signal and detecting an overshoot and/or undershoot of the dynamic range is repeated with a respective adjusted setting of the analog signal processing unit, until a predetermined termination criteria is satisfied, and wherein the correction of the digital signal occurs according to the last setting of the analog signal processing unit.

9. A computer implemented method for controlling an optical coherence tomography measurement, comprising
- acquiring a digital signal of a measurement depth-dependent backscattering profile of an optical coherence tomography measurement generated by a signal converter unit;
- detecting an overshoot and/or undershoot of a dynamic range of the signal converter unit;
- generating a control signal for controlling an adaptive analog signal processing unit such that a selective weighting, caused by the adaptive analog signal processing unit of analog signal portions belonging to different measurement depths of the backscattering profile of the optical coherence tomography measurement of the detected overshoot and/or undershoot of the dynamic range is counteracted; and
- correcting the digital signal in such a manner that thereby the selective weighting of different signal portions performed by the signal processing unit is compensated.

10. A computer system for controlling an optical coherence tomography measurement, comprising
- a digital signal input for acquiring a digital signal of a measurement depth-dependent backscattering profile of an optical coherence tomography measurement generated by a signal converter unit;
- a data evaluation module for detecting an overshoot and/or undershoot of a dynamic range of the signal converter unit;
- a data memory with a control rule which for overshoots and/or undershoots of the dynamic range of the signal converter unit determines values of a control signal for controlling an adaptive analog signal processing unit in such a manner that a selective weighting of analog signal portions belonging to different measurement depths of the backscattering profile of the optical coherence tomography measurement caused by the control signal from the adaptive analog signal processing unit counteracts the overshoots and/or undershoots of the dynamic range; and
- a control module, which generates a control signal of the detected overshoot and/or undershoot of the dynamic range according to the control regulation stored in the data memory,
wherein the data evaluation module is designed to correct the digital signal such that thereby the selective weighting of different signal portions caused by the signal processing unit according to the control rule for the generated control signal, is compensated.

## Revendications

1. Système de tomographie en cohérence optique comprenant :
- un module de détection optique (200, 201, 209) pour la détection optique d'un profil de rétrodiffusion optique dépendant de la profondeur de mesure (110), dans lequel le module de détection optique (200, 201, 209 ; 601, 602, 209) est conçu pour générer un signal de mesure analogique (216) du profil de rétrodiffusion optique dépendant de la profondeur de mesure détecté (110), dans lequel le module de détection optique comprend un module de traitement de signal analogique pouvant être commandé (208) qui est conçu pour effectuer en fonction du signal de commande détecté (217) une pondération sélective des portions du signal de mesure analogique appartenant aux différentes profondeurs de mesure ;
- un module convertisseur de signal (211) qui est conçu pour détecter le signal de mesure analogique (216) du module de détection optique et le transformer en un signal numérique ; et
- un module de détection et de traitement de données (210) qui est conçu pour déterminer des dépassements et/ou dépassements par le bas d'une plage dynamique du module convertisseur de signal (211), commander le module de traitement de signal analogique au moyen du signal de commande de telle sorte qu'un dépassement et/ou dépassement par le bas déterminé de la plage dynamique du module convertisseur de signal est contrecarré et effectuer une correction du signal numérique de telle sorte que la pondération effectuée par le module de traitement de signal de différentes portions de signal est compensée de ce fait.

2. Système selon la revendication 1, dans lequel le module de détection optique comprend :
- une source de lumière (601) dont la longueur d'onde est variable dans le temps au sein d'une plage de balayage du module de détection optique ; et
- un détecteur optique (209) qui est conçu pour transformer un signal optique du module de détection optique en un signal de mesure électrique analogique (604), dans lequel le module de traitement de signal analogique (208) comprend un filtre invariant dans le temps, linéaire adaptatif qui effectue un filtrage du signal de mesure électrique.

3. Système selon la revendication 2, dans lequel le filtre adaptatif comprend un filtre passe-haut et/ou un filtre passe-bande et/ou un filtre coupe-bande et/ou un module d'amplification à large bande.

4. Système selon la revendication 1, dans lequel le module de détection optique comprend un interféromètre optique (200) avec un bras d'échantillonnage (204) et un bras de référence (203), dans lequel une différence des chemins optiques entre le bras d'échantillonnage (204) et le bras de référence (203) est variable dans le temps pour le balayage de la profondeur de mesure, et dans lequel le module de traitement de signal analogique (208) comprend un organe d'amplification et/ou d'amortissement linéaire variable dans le temps qui est commandé par le module de détection et de traitement de données (210) en fonction de la différence des chemins optiques entre le bras d'échantillonnage (204) et le bras de référence (203).

5. Système selon la revendication 4, dans lequel l'organe d'amplification et/ou d'amortissement comprend un organe d'amplification et/ou d'amortissement électrique.

6. Système selon la revendication 4, dans lequel l'organe d'amplification et/ou d'amortissement comprend un organe d'amplification et/ou d'amortissement optique.

7. Procédé de tomographie en cohérence optique comprenant :
- détection d'un profil de rétrodiffusion optique dépendant de la profondeur de mesure au moyen d'un module de détection optique qui génère un signal de mesure analogique ;
- transformation du signal de mesure analogique en un signal numérique au moyen d'un module convertisseur de signal (211) ;
- détermination d'un dépassement et/ou dépassement par le bas d'une plage dynamique du module convertisseur de signal (211) ;
- pondération sélective des portions du signal de mesure analogique appartenant aux différentes profondeurs de mesure au moyen d'un module de traitement de signal analogique adaptatif (208) de telle sorte qu'un dépassement et/ou dépassement par le bas déterminé de la plage dynamique est contrecarré ;
- correction du signal numérique de telle sorte que la pondération effectuée au moyen du module de traitement de signal analogique de différentes portions de signal analogique est compensée de ce fait.

8. Procédé selon la revendication 7, dans lequel la détection du profil de rétrodiffusion optique, la génération d'un signal de mesure analogique et la détermination d'un dépassement et/ou dépassement par le bas de la plage dynamique sont d'abord réalisés pour un premier réglage du module de traitement de signal analogique adaptatif, dans lequel un réglage adapté du module de traitement de signal analogique adaptatif est ensuite effectué en fonction du dépassement et/ou dépassement par le bas détecté de la plage dynamique, et dans lequel la détection du profil de rétrodiffusion optique, la génération d'un signal de mesure analogique et la détermination d'un dépassement et/ou dépassement par le bas de la plage dynamique sont répétés dans le cas d'un réglage adapté à chaque fois du module de traitement de signal analogique jusqu'à ce qu'un critère d'interruption prédéterminé soit satisfait, et dans lequel la correction du signal numérique s'effectue selon le dernier réglage du module de traitement de signal analogique.

9. Procédé mis en oeuvre par ordinateur pour la commande d'une mesure de tomographie en cohérence optique comprenant
- détection d'un signal numérique généré par un module convertisseur de signal d'un profil de rétrodiffusion dépendant de la profondeur de mesure d'une mesure de tomographie en cohérence optique ;
- détermination d'un dépassement et/ou dépassement par le bas d'une plage dynamique du module convertisseur de signal ;
- génération d'un signal de commande pour la commande d'un module de traitement de signal analogique adaptatif de telle sorte qu'une pondération sélective entraînée par le signal de commande du module de traitement de signal analogique adaptatif de portions de signal analogique appartenant aux différentes profondeurs de mesure du profil de rétrodiffusion de la mesure de tomographie en cohérence optique contrecarre le dépassement et/ou dépassement par le bas déterminé de la plage dynamique ; et
- correction du signal numérique de telle sorte que la pondération sélective effectuée par le module de traitement de signal de différentes portions de signal est compensée de ce fait.

10. Système informatique pour la commande d'une mesure de tomographie en cohérence optique comprenant
- une entrée de signal numérique pour la détection d'un signal numérique généré par un module convertisseur de signal d'un profil de rétrodiffusion dépendant de la profondeur de mesure d'une mesure de tomographie en cohérence optique ;
- un module d'évaluation de données pour la détermination d'un dépassement et/ou dépassement par le bas d'une plage dynamique du module convertisseur de signal ;
- une mémoire de données avec une instruction de commande qui définit pour des dépassements et/ou dépassements par le bas de la plage dynamique du module convertisseur de signal des valeurs d'un signal de commande pour la commande d'un module de traitement de signal analogique adaptatif de telle sorte qu'une pondération sélective entraînée par le signal de commande du module de traitement de signal analogique adaptatif de portions de signal analogique appartenant aux différentes profondeurs de mesure du profil de rétrodiffusion de la mesure de tomographie en cohérence optique contrecarre les dépassements et/ou dépassements par le bas de la plage dynamique ; et
- un module de commande qui génère un signal de commande le dépassement et/ou dépassement par le bas déterminé de la plage dynamique conformément à l'instruction de commande déposée dans la mémoire de données,
dans lequel le module d'évaluation de données est conçu pour corriger le signal numérique de telle sorte que la pondération sélective entraînée par le module de traitement de signal conformément à l'instruction de commande pour le signal de commande généré de différentes portions de signal est compensée de ce fait.
